# EUROPEAN PATENT SPECIFICATION

(11) **EP 4 262 562 B1**
(45) Date of publication and mention of the grant of the patent: **18.02.2026**
(21) Application number: 21854727.1
(22) Date of filing: 10.12.2021
(51) Int. Cl.: A61B 7/00, A61B 5/08

(54) **HEAD-WEARABLE APPARATUS FOR BREATHING ANALYSIS**
AM KOPF TRAGBARE VORRICHTUNG ZUR ATEMANALYSE
APPAREIL À PORTER SUR LA TÊTE POUR ANALYSE RESPIRATOIRE

(30) Priority: 18.12.2020 US 202063127855 P; 22.11.2021 US 202117456096
(43) Date of publication of application: 25.10.2023
(73) Proprietor: Snap Inc., Santa Monica, CA 90405 (US)
(72) Inventor: ARYA, Ashwani, Santa Monica, California 90405 (US); BAHULKAR, Tejas, Santa Monica, California 90405 (US)
(74) Representative: Kramer, Dani
(86) International application number: PCT/US2021/072861
(87) International publication number: WO 2022/133410

(56) References cited:
- WO-A1-2021/198941
- CN-A- 111 696 575
- US-A1- 2019 038 216
- US-A1- 2021 290 105
- WANG ANRAN ANRANW@UW EDU ET AL: "Contactless Infant Monitoring using White Noise", MOBILE COMPUTING AND NETWORKING, ACM, 2 PENN PLAZA, SUITE 701NEW YORKNY10121-0701USA, 11 October 2019 (2019-10-11), pages 1 - 16, XP058475525, ISBN: 978-1-4503-6169-9, DOI: 10.1145/3300061.3345453

## Description

### CLAIM OF PRIORITY

This application claims the benefit of priority to U.S. Provisional Application Serial No. 63/127,855, filed on December 18, 2020 and U.S. Patent Application Serial No. 17/456,096, filed on November 22, 2021.

### BACKGROUND

Currently, a user can use the built-in camera(s) on a mobile device to quickly capture an event or moment occurring in the user's life. A number of head-worn devices are adapted to capture audio and/or visual content. For example, so-called smart glasses can capture audio and/or visual (A/V) content without the user having to take out their mobile device. A/V content captured in this manner can be shared with other people over a messaging application or social networking applications.

Smart glasses may also provide additional functionality to the user. For example, AR (augmented reality) smart glasses can provide visual or audio information to supplement the user's real-word experience. Prior art documents CN 111 696 575 and US 2019/038216 both disclose the preambles of independent claims 1, 8 and 13.

### BRIEF DESCRIPTION OF THE DRAWINGS

In the drawings, which are not necessarily drawn to scale, like numerals may describe similar components in different views. To easily identify the discussion of any particular element or act, the most significant digit or digits in a reference number refer to the figure number in which that element is first introduced. Some nonlimiting examples are illustrated in the figures of the accompanying drawings in which:
FIG. 1 illustrates a perspective view of a head-wearable apparatus according to one example.
FIG. 2 illustrates a bottom view of the head-wearable apparatus of FIG. 1, according to one example.
FIG. 3 is a diagrammatic representation of a networked environment in which the present disclosure may be deployed, in accordance with some examples.
FIG. 4 is a block diagram of an audio processor for use with the head-wearable apparatus of FIG. 1 according to one example.
FIG. 5 illustrates a block diagram of the beamformer of FIG. 4, according to one example.
FIG. 6 is a graph of an example of a signal received by the breath signal analysis module of FIG. 4.
FIG. 7A is a graph showing an example of an audio frequency distribution of an inhale.
FIG. 7B shows an example of an audio frequency distribution of a corresponding exhale.
FIG. 8 is a flow diagram of a breath monitoring process using the head-wearable apparatus of FIG. 1 according to one example.
FIG. 9 illustrates an aspect of the subject matter in accordance with one embodiment.
FIG. 10 illustrates an aspect of the subject matter in accordance with one embodiment.
FIG. 11 is a block diagram of an audio processor for use with the head-wearable apparatus of FIG. 1 according to one example.
FIG. 12 is a flow diagram of a breath monitoring process using the head-wearable apparatus of FIG. 1 according to one example.
FIG. 13 is a diagrammatic representation of a machine in the form of a computer system within which a set of instructions may be executed for causing the machine to perform any one or more of the methodologies discussed herein, in accordance with some examples.
FIG. 14 is a block diagram showing a software architecture within which examples may be implemented.

### DETAILED DESCRIPTION

The invention and preferred embodiments thereof is defined in the appended claims. The description that follows includes systems, methods, techniques, instruction sequences, and computing machine program products that embody illustrative examples of the disclosure. In the following description, for the purposes of explanation, numerous specific details are set forth in order to provide an understanding of various examples of the inventive subject matter. It will be evident, however, to those skilled in the art, that examples of the inventive subject matter may be practiced without these specific details. In general, well-known instruction instances, protocols, structures, and techniques are not necessarily shown in detail.

Breathing exercises or mindful breathing techniques may provide a number of physical and mental benefits, including for example stress reduction, lower blood pressure, etc. In this regard, it may be beneficial to provide monitoring and feedback to a person who is doing breathing exercises.

In one example, a head wearable apparatus comprises a pair of spectacles including one or more spaced apart microphones. The spectacles may for example be so-called smart glasses and the microphones may otherwise be used for voice calls or to capture ambient audio to accompany video captured by one or more cameras included in the spectacles. The signal from the microphones may be beamformed to better detect breathing sounds. Various breath-related parameters can then be determined from the beamformed signal to provide feedback to the person doing the breathing exercises.

The invention as defined in independent claim 1 relates to a method of monitoring the breathing of a user performed by one or more processors, the method comprising receiving a plurality of audio signals from a plurality of microphones, beamforming the plurality of audio signals into a beamformed audio signal, determining observed breath-related parameters from the beamformed audio signal, and providing an output of or derived from the observed breath-related parameters.

The method further comprises determining a nature of the breathing or a state of the user from the observed breath-related parameters using a trained machine learning model, wherein the output relates to the nature of the breathing or a state of the user. The method defined in independent claim 1 further comprises requesting feedback from the user on the nature of the breathing or a state of the user, receiving feedback from the user on the nature of the breathing or the state of the user; and updating the trained machine learning model based in the feedback received from the user. The breath metrics may be determined by comparing the observed breath-related parameters with reference breath-related parameters, and the reference breath-related parameters may be historical breath-related parameters for the user. The observed breath-related parameters may include frequency characteristics of an inhale or an exhale.

A breathing score may be derived from the breath metrics. The breathing score may be an accuracy score based on how closely an observed respiration of the user matches a prompted or desired respiration rate. The breathing score may be an accuracy score based on a combination of how closely an observed respiration rate of the user matches a prompted or desired respiration rate and an observed amount of time. The observed amount of time is an amount of time that the observed respiration rate is within a threshold value of the prompted or desired respiration rate.

A user may provide an input indicating that the breathing score is to be shared with one or more recipients, and in response a message including the breathing score may be transmitted over a network.

The invention as defined in independent claim 8 relates to a non-transitory machine-readable storage medium comprising instructions that, when executed by one or more processors of a machine, cause the machine to perform operations for monitoring the breathing of a user, comprising receiving a plurality of audio signals from a plurality of microphones, beamforming the plurality of audio signals into a beamformed audio signal, determining observed breath-related parameters from the beamformed audio signal, and providing an output of or derived from the observed breath-related parameters.

The operations further comprise determining a nature of the breathing or a state of the user from the observed breath-related parameters using a trained machine learning model, wherein the output comprises the nature of the breathing or a state of the user. The operations of claim 8 further comprise: requesting feedback from the user on the nature of the breathing or a state of the user, receiving feedback from the user on the nature of the breathing or the state of the user; and updating the trained machine learning model based in the feedback received from the user. The breath metrics may be determined by comparing the observed breath-related parameters with reference breath-related parameters.

Thee operations may further comprise deriving a breathing score from the breath metrics, wherein the breathing score is an accuracy score based on how closely an observed respiration rate of the user matches a prompted or desired respiration rate. The breathing score may further be based on an observed amount of time that the observed respiration rate is within a threshold value of the prompted or desired respiration rate.

The invention as defined in independent claim 13 relates to a system comprising one or more processors and one or more machine-readable mediums storing instructions that, when executed by the one or more processors, cause the system to perform operations for monitoring the breathing of a user as described herein. The operations comprise receiving a plurality of audio signals from a plurality of microphones, beamforming the plurality of audio signals into a beamformed audio signal, determining observed breath-related parameters from the beamformed audio signal, wherein said step of determining breath-related parameters comprises determining a nature of the breathing or a state of the user from the observed breath-related parameters using a trained machine learning model, and providing an output to the user of the nature of the breathing or a state of the user. nature of the breathing or a state of the user. The operations of claim 13 further comprise: requesting feedback from the user on the nature of the breathing or a state of the user, receiving feedback from the user on the nature of the breathing or the state of the user; and updating the trained machine learning model based in the feedback received from the user.

The operations may further comprise determining breath metrics from the observed breath-related parameters by comparing the observed breath-related parameters with reference breath-related parameters, and wherein the output comprises the breath metrics. The observed breath-related parameters may include frequency characteristics of an inhale or an exhale.

The operations may further comprise deriving a breathing score from the breath metrics, the breathing score being based on a combination of how closely an observed respiration of the user matches a prompted or desired respiration rate and an observed amount of time that the observed respiration rate is within a threshold value of the prompted or desired respiration rate.

The operations may also further comprise receiving user input indicating that the breathing score is to be shared with one or more recipients, and transmitting a message including the breathing score over a network.

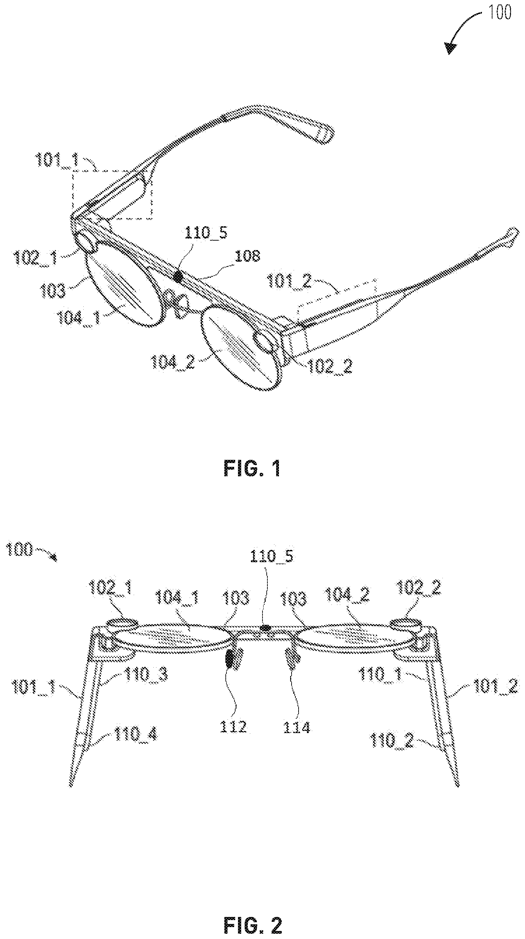

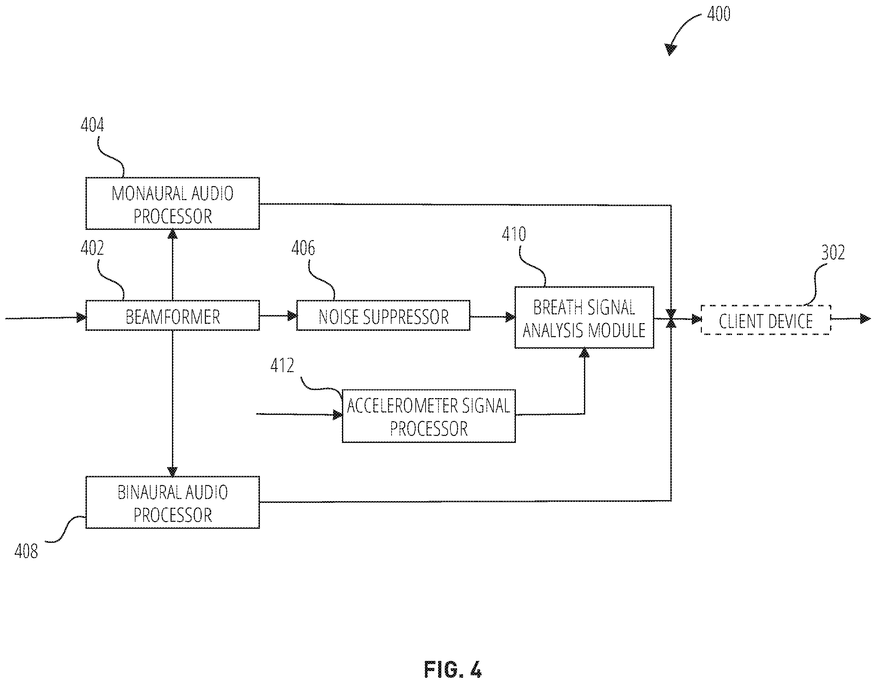

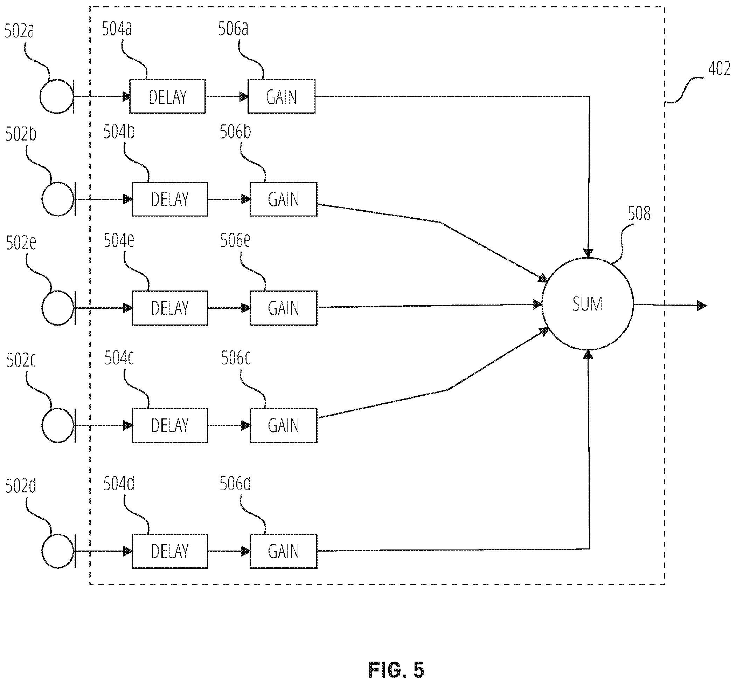

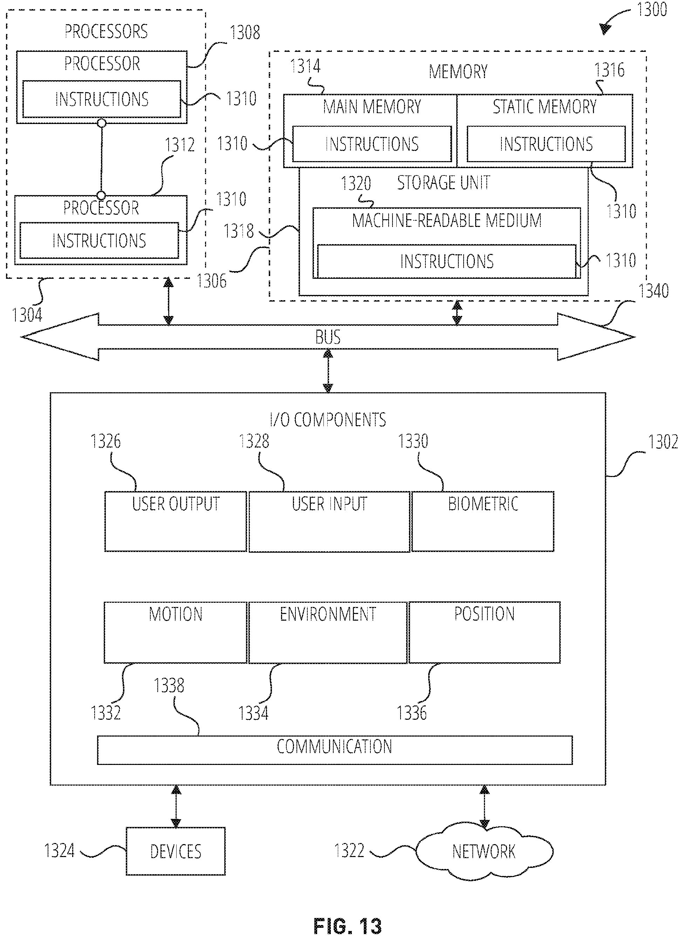

FIG. 1 illustrates a perspective view of a head-wearable apparatus 100 to monitor breathing according to one example. FIG. 2 illustrates a bottom view of the head-wearable apparatus 100 from FIG. 1, according to one example. In FIG. 1 and FIG. 2, the head-wearable apparatus 100 is a pair of eyeglasses. In some examples, the head-wearable apparatus 100 can be sunglasses or goggles. Some examples can include one or more wearable devices, such as a pendant with an integrated camera that is integrated with, in communication with, or coupled to, the head-wearable apparatus 100 or a client device. Any desired wearable device may be used in conjunction with the examples of the present disclosure, such as a watch, a headset, a wristband, earbuds, clothing (such as a hat or jacket with integrated electronics), a clip-on electronic device, or any other wearable devices. It is understood that, while not shown, one or more portions of the system included in the head-wearable apparatus can be included in a client device (e.g., machine 1300 of FIG. 13) that can be used in conjunction with the head-wearable apparatus 100. For example, one or more elements as shown in FIG. 4 and FIG. 5 can be included in the head-wearable apparatus 100 and/or the client device.

As used herein, the term "client device" may refer to any machine that interfaces to a communications network to obtain resources from one or more server systems or other client devices. A client device may be, but is not limited to, a mobile phone, desktop computer, laptop, portable digital assistants (PDAs), smart phones, tablets, ultra books, netbooks, laptops, multi-processor systems, microprocessor-based or programmable consumer electronics, game consoles, set-top boxes, or any other communication device that a user may use to access a network.

In FIG. 1 and FIG. 2, the head-wearable apparatus 100 is a pair of eyeglasses that includes a frame 103 that includes eye wires (or rims) that are coupled to two stems (or temples), respectively, via hinges and/or end pieces. The eye wires of the frame 103 carry or hold a pair of lenses 104_1, 104_2. The frame 103 includes a first (e.g., right) side that is coupled to the first stem and a second (e.g., left) side that is coupled to the second stem. The first side is opposite the second side of the frame 103.

The apparatus 100 further includes a camera module that includes camera lenses 102_1, 102_2 and at least one image sensor. The camera lens may be a perspective camera lens or a non-perspective camera lens. A non-perspective camera lens may be, for example, a fisheye lens, a wide-angle lens, an omnidirectional lens, etc. The image sensor captures digital video through the camera lens. The images may be also be still image frame or a video including a plurality of still image frames. The camera module can be coupled to the frame 103. As shown in FIG. 1 and FIG. 2, the frame 103 is coupled to the camera lenses 102_1, 102_2 such that the camera lenses face forward. The camera lenses 102_1, 102_2 can be perpendicular to the lenses 104_1, 104_2. The camera module can include dual-front facing cameras that are separated by the width of the frame 103 or the width of the head of the user of the apparatus 100.

In FIG. 1 and FIG. 2, the two stems (or temples) are respectively coupled to microphone housings 101_1, 101_2. The first and second stems are coupled to opposite sides of the frame 103 of the head-wearable apparatus 100. The first stem is coupled to the first microphone housing 101_1 and the second stem is coupled to the second microphone housing 101_2. The microphone housings 101_1, 101_2 can be coupled to the stems between the locations of the frame 103 and the temple tips. The microphone housings 101_1, 101_2 can be located on either side of the user's temples when the user is wearing the apparatus 100.

In the example shown in FIG.2, the first microphone housing 101_1 encases microphones 110_3 and 110_4 and the second microphone housing 101_2 encases microphones 110_1 and 110_2. Each of the microphone housings 101_1, 101_2 includes a front port and a rear port. The front port of the first microphone housing 101_1 is coupled to microphone 110_3 (e.g. first front microphone) and the rear port of the first microphone housing 101_1 is coupled to the microphone 110_4 (e.g., first rear microphone). The front port of the second microphone housing 101_2 is coupled to microphone 110_1 (e.g. second front microphone) and the rear port of the second microphone housing 101_2 is coupled to the microphone 110_2 (e.g., second rear microphone).

Also included in the example shown in FIG. 2 is a microphone 101_5 located in a bridge 108 of the eyeglasses above the nosepiece 114. The microphone 101_5 may be provided in addition to microphones 101_1 to 101_4 to provide an additional signal corresponding to the user's breathing sounds or vocals.

Microphones 110_1 to 110_N may be digital or analog micro electro-mechanical systems (MEMS) microphones.

Additionally, the head-wearable apparatus 100 may include one or more accelerometers. The accelerometers may be special purpose accelerometers for use in breath monitoring, or they could be part of an inertial measurement unit (IMU) such as the motion components 1332 described with reference to FIG. 13.

For example, FIG. 2 shows an accelerometer 112 mounted to a nosepiece 114 of the head-wearable apparatus 100. It will be appreciated that other suitable locations for one or more accelerometers may be selected, including in the bridge 108 of the eyeglasses above the nosepiece 114. Additionally, the IMU may also be located in or around the nosepiece 114 or bridge 108. In such a case the configuration of the head-wearable apparatus 100 may be modified, for example to include an integrated bridge and nosepiece, or otherwise as necessary.

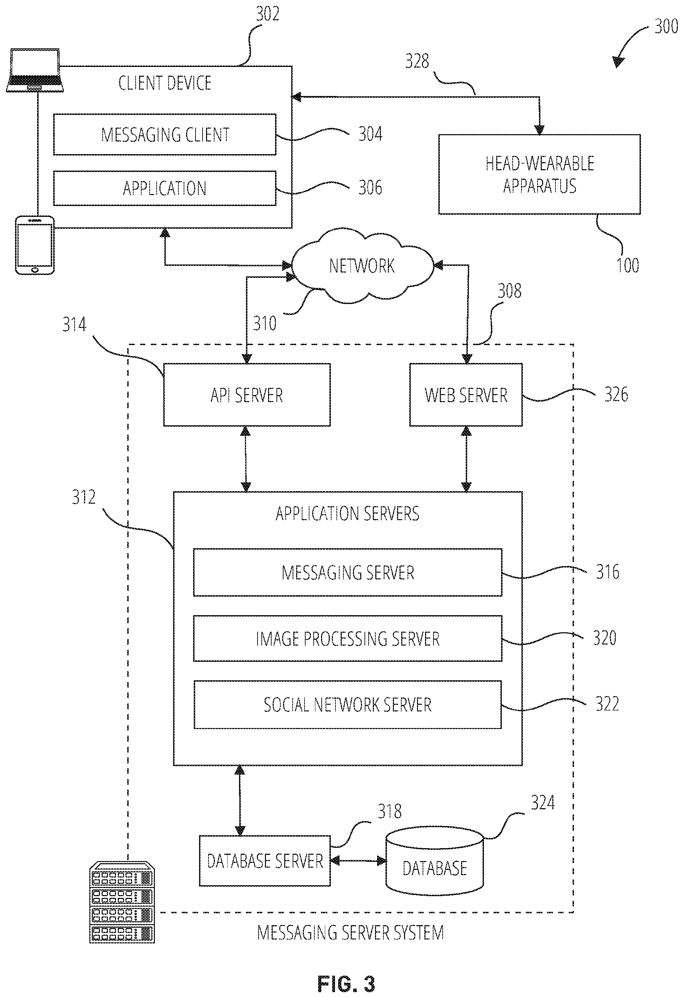

FIG. 3 is a block diagram showing an example messaging system 300 for exchanging data (e.g., messages and associated content) over a network. The messaging system 300 includes multiple instances of a client device 302, each of which hosts a number of applications, including a messaging client 304 and other applications 306. Each messaging client 304 is communicatively coupled to other instances of the messaging client 304 (e.g., hosted on respective other client devices 302) and a messaging server system 308 via a network 310 (e.g., the Internet). A messaging client 304 can also communicate with locally-hosted applications 306 using Applications Program Interfaces (APIs).

As can be seen, the head-wearable apparatus 100 may be included in the networked computing environment of FIG. 3. by means of a data link 328 that couples the head-wearable apparatus 100 to the client device 302. In one example, the data link 328 is a wireless data link operating on a short-range protocol such as Bluetooth. The data link is operable to provide the exchange of data (e.g. audio and video data) and commands or user inputs between head-wearable apparatus 100 and client device 302

A messaging client 304 is able to communicate and exchange data with other messaging clients 304 and with the messaging server system 308 via the network 310. The data exchanged between messaging clients 304, and between a messaging client 304 and the messaging server system 308, includes functions (e.g., commands to invoke functions) as well as payload data (e.g., text, audio, video or other multimedia data).

The messaging server system 308 provides server-side functionality via the network 310 to a particular messaging client 304. While certain functions of the messaging system 300 are described herein as being performed by either a messaging client 304 or by the messaging server system 308, the location of certain functionality either within the messaging client 304 or the messaging server system 308 may be a design choice. For example, it may be technically preferable to initially deploy certain technology and functionality within the messaging server system 308 but to later migrate this technology and functionality to the messaging client 304 where a client device 302 has sufficient processing capacity.

The messaging server system 308 supports various services and operations that are provided to the messaging client 304. Such operations include transmitting data to, receiving data from, and processing data generated by the messaging client 304. This data may include message content, client device information, geolocation information, media augmentation and overlays, message content persistence conditions, social network information, and live event information, as examples. Data exchanges within the messaging system 300 are invoked and controlled through functions available via user interfaces (UIs) of the messaging client 304.

Turning now specifically to the messaging server system 308, an Application Program Interface (API) server 314 is coupled to, and provides a programmatic interface to, application servers 312. The application servers 312 are communicatively coupled to a database server 318, which facilitates access to a database 324 that stores data associated with messages processed by the application servers 312. Similarly, a web server 326 is coupled to the application servers 312, and provides web-based interfaces to the application servers 312. To this end, the web server 326 processes incoming network requests over the Hypertext Transfer Protocol (HTTP) and several other related protocols.

The Application Program Interface (API) server 314 receives and transmits message data (e.g., commands and message payloads) between the client device 302 and the application servers 312. Specifically, the Application Program Interface (API) server 314 provides a set of interfaces (e.g., routines and protocols) that can be called or queried by the messaging client 304 in order to invoke functionality of the application servers 312. The Application Program Interface (API) server 314 exposes various functions supported by the application servers 312, including account registration, login functionality, the sending of messages, via the application servers 312, from a particular messaging client 304 to another messaging client 304, the sending of media files (e.g., images or video) from a messaging client 304 to a messaging server 316, and for possible access by another messaging client 304, the settings of a collection of media data (e.g., story), the retrieval of a list of friends of a user of a client device 302, the retrieval of such collections, the retrieval of messages and content, the addition and deletion of entities (e.g., friends) to an entity graph (e.g., a social graph), the location of friends within a social graph, and opening an application event (e.g., relating to the messaging client 304).

The application servers 312 host a number of server applications and subsystems, including for example a messaging server 316, an image processing server 320, and a social network server 322. The messaging server 316 implements a number of message processing technologies and functions, particularly related to the aggregation and other processing of content (e.g., textual and multimedia content) included in messages received from multiple instances of the messaging client 304. As will be described in further detail, the text and media content from multiple sources may be aggregated into collections of content (e.g., called stories or galleries). These collections are then made available to the messaging client 304. Other processor and memory intensive processing of data may also be performed server-side by the messaging server 316, in view of the hardware requirements for such processing.

The application servers 312 also include an image processing server 320 that is dedicated to performing various image processing operations, typically with respect to images or video within the payload of a message sent from or received at the messaging server 316.

The social network server 322 supports various social networking functions and services and makes these functions and services available to the messaging server 316. Examples of functions and services supported by the social network server 322 include the identification of other users of the messaging system 300 with which a particular user has relationships or is "following," and also the identification of other entities and interests of a particular user.

Returning to the messaging client 304, features and functions of an external resource (e.g., an application 306 or applet) are made available to a user via an interface of the messaging client 304. In this context, "external" refers to the fact that the application 306 or applet is external to the messaging client 304. The external resource is often provided by a third party but may also be provided by the creator or provider of the messaging client 304. The messaging client 304 receives a user selection of an option to launch or access features of such an external resource. The external resource may be the application 306 installed on the client device 302 (e.g., a "native app"), or a small-scale version of the application (e.g., an "applet") that is hosted on the client device 302 or remote of the client device 302 (e.g., on a third-party server). The small-scale version of the application includes a subset of features and functions of the application (e.g., the full-scale, native version of the application) and is implemented using a markup-language document. In one example, the small-scale version of the application (e.g., an "applet") is a web-based, markup-language version of the application and is embedded in the messaging client 304. In addition to using markup-language documents (e.g., a . *ml file), an applet may incorporate a scripting language (e.g., a .*js file or a .json file) and a style sheet (e.g., a .*ss file).

In response to receiving a user selection of the option to launch or access features of the external resource, the messaging client 304 determines whether the selected external resource is a web-based external resource or a locally-installed application 306. In some cases, applications 306 that are locally installed on the client device 302 can be launched independently of and separately from the messaging client 304, such as by selecting an icon, corresponding to the application 306, on a home screen of the client device 302. Small-scale versions of such applications can be launched or accessed via the messaging client 304 and, in some examples, no or limited portions of the small-scale application can be accessed outside of the messaging client 304. The small-scale application can be launched by the messaging client 304 receiving, from a third-party server for example, a markup-language document associated with the small-scale application and processing such a document.

In response to determining that the external resource is a locally-installed application 306, the messaging client 304 instructs the client device 302 to launch the external resource by executing locally-stored code corresponding to the external resource. In response to determining that the external resource is a web-based resource, the messaging client 304 communicates with third-party servers (for example) to obtain a markup-language document corresponding to the selected external resource. The messaging client 304 then processes the obtained markup-language document to present the web-based external resource within a user interface of the messaging client 304.

The messaging client 304 can notify a user of the client device 302, or other users related to such a user (e.g., "friends"), of activity taking place in one or more external resources. For example, the messaging client 304 can provide participants in a conversation (e.g., a chat session) in the messaging client 304 with notifications relating to the current or recent use of an external resource by one or more members of a group of users. One or more users can be invited to join in an active external resource or to launch a recently-used but currently inactive (in the group of friends) external resource. The external resource can provide participants in a conversation, each using respective messaging clients 304, with the ability to share an item, status, state, or location in an external resource with one or more members of a group of users into a chat session. The shared item may be an interactive chat card with which members of the chat can interact, for example, to launch the corresponding external resource, view specific information within the external resource, or take the member of the chat to a specific location or state within the external resource. Within a given external resource, response messages can be sent to users on the messaging client 304. The external resource can selectively include different media items in the responses, based on a current context of the external resource.

The messaging client 304 can present a list of the available external resources (e.g., applications 306 or applets) to a user to launch or access a given external resource. This list can be presented in a context-sensitive menu. For example, the icons representing different ones of the application 306 (or applets) can vary based on how the menu is launched by the user (e.g., from a conversation interface or from a non-conversation interface).

FIG. 4 illustrates a block diagram of an audio processor 400 for use with head-wearable apparatus 100 according to one example. The audio processor 400 may be implemented in the head-wearable apparatus 100 or in an associated computing device such as client device 302. One embodiment of a client device 302 is machine 1300 discussed in more detail below with reference to FIG. 13. The audio processor 400 in one example includes a beamformer 402, a monaural audio processor 404, a binaural audio processor 408, a noise suppressor 406, and a breath signal analysis module 410. The outputs of the monaural audio processor 404, binaural audio processor 408 breath signal analysis module 410 are provided to the client device 302 if one or more of these elements are not embodied in the client device 302 itself.

The beamformer 402 receives audio signals from the microphones 110_1 to 110_N and combines them in such a way that acoustic signals at locations of interest experience constructive interference while others experience destructive interference. This permits enhancement of the resulting audio output signal from the beamformer 402. The beamformer 402 in the example of FIG. 4 has different modes depending on the mode of operation of the audio processor 400, for example when used for generating a binaural audio signal (e.g. for video capture) versus a monaural audio signal, (e.g. for a telephone call or for breath analysis). The beamformer 402 is illustrated in more detail in FIG. 5.

The monaural audio processor 404 is configured to provide, in one example, output corresponding to the speech of a user, for use in speech communications with a remote device or to capture a user's speech for other purposes, such as for speech to text conversion for a messaging application or when saving an audio memo to local or remote storage. The monaural audio processor 404 provides conventional speech audio processing as is known in the art.

The binaural audio processor 408 is configured to provide, in one example, binaural output corresponding to environmental sounds in the vicinity of the head-wearable apparatus 100, for example when the user is capturing video using the head-wearable apparatus 100. The binaural audio processor generates a signal corresponding to the audio ahead of the head-wearable apparatus 100 that relates generally to the video being captured. In one example, the binaural audio processor provides the processing described in issued US patent no. 10,567,898, the contents of which are incorporated herein, but it will be appreciated that other binaural processing techniques known in the art may also be used.

The noise suppressor 406 and breath signal analysis module 410 are configured to generate data corresponding to breathing sounds generated by a user of the head-wearable apparatus 100 during respiration. The noise suppressor 406 uses one or more active or passive noise reduction techniques known in the art. The breath signal analysis module 410 processes an output signal from the noise suppressor 406 to extract data from the user's breathing sounds as will be discussed in more detail below.

The audio processor 400 is able to switch between different modes depending on user input or automatically by the user device, depending on context. For example, if a call is received or initiated on the client device 302, or a voice recognition mode is activated on the user device, the parameters of the beamformer 402 may be set to provide output signals appropriate for such processing by the monaural audio processor 404, while if video capture is enabled on for the head-wearable apparatus 100, the beamformer 402 may be set to provide output signals appropriate for such processing by the binaural audio processor 408. Similarly, if user input corresponding to breath monitoring is detected, or breath monitoring is otherwise enabled on the client device 302, the beamformer 402 may be set to provide output signals appropriate for processing by the breath signal analysis module 410.

It will also be appreciated that while three signal paths are shown in FIG. 4 for processing audio from the microphones, the audio processor 400 may also be implemented as a single adaptive audio path to provide the required processing. Additionally, more than one beamformer 402 may be included in the audio processor 400, to provide enhancement of signals at more than one spatial location, such as in a binaural implementation.

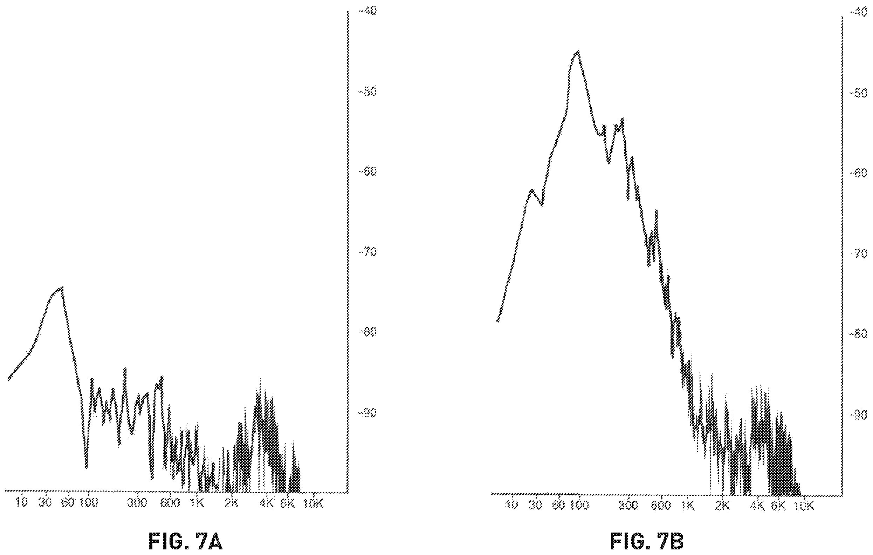

In the case in which an accelerometer input signal is provided from, for example the accelerometer 112 or from motion components 1332, the accelerometer input signal is first amplified and filtered at accelerometer signal processor 412. The amplification and filtering are selected to provide an output from accelerometer signal processor 412 that is comparable to the audio signal received by the breath signal analysis module 410 from the noise suppressor 406. This output is then provided to the breath signal analysis module 410. The output from the accelerometer signal processor 412 may be processed by the breath signal analysis module 410 in the same manner as the output from the noise suppressor 406 as discussed in more detail below with reference to FIG. 7A and FIG. 7B. The parameters and metrics derived from the accelerometer signal may be used in the event the audio signal is weak and also to do cross verification in the event there is noise arising from rubbing sounds on the microphone(s). In one embodiment, the breath parameters and metrics may be derived solely from the accelerometer output.

The outputs of monaural audio processor 404, breath signal analysis module 410, binaural audio processor 408 are provided to client device 302 (if audio processor 400 is not embodied in client device 302) over data link 328 for provision to the messaging client 304 or the application 306, or are provided to the messaging client 304 or application 306 if the audio processor 400 is embodied in the client device 302. It will also be appreciated that various functions or modules of the audio processor 400 may be distributed between the head-wearable apparatus 100 and the client device 302.

FIG. 5 illustrates a block diagram of the beamformer 402 of FIG. 4, according to one example.

Beamformer 402 includes a plurality of delays 504a to 404e, a plurality of gains 506a to 406e and a summing junction 508. The beamformer 402 is coupled to microphones 502a to 402e corresponding to microphones 110_1 to 110_5 respectively. Each delay (e.g. delay 504a) receives an input signal from a corresponding microphone (e.g. microphone 502a) and applies a time delay to the input signal. The time delay is settable by the audio processor 400 (e.g. under instruction of the client device 302) to steer the beamformer 402 as is known in the art. In some cases the time delay, for example for 502e (corresponding to microphone 110_5), may be set to zero since this microphone would be the central microphone in an array that is being used to capture audio (breathing sounds) from sources (the user's mouth and nose) that are located on a plane of symmetry.

Each delay (e.g. delay 504a) provides an output signal to a corresponding gain (e.g. gain 506a), which applies a variable gain to each output signal. The gain value applied by each gain (e.g. gain 506a) is settable by the audio processor 400 (e.g. under instruction of the client device 302) to normalize signals from the microphones 502a to 402e as is known in the art. In some cases the gain, for example for 502e (corresponding to microphone 110_5). may be set to zero if the audio processor 400 is operating in a mode, for example a binaural mode, in which 502e is not used.

The outputs of each of the gains is provided to a summing junction 508, where it is combined into an output signal that is provided to monaural audio processor 404, noise suppressor 406, or binaural audio processor 408.

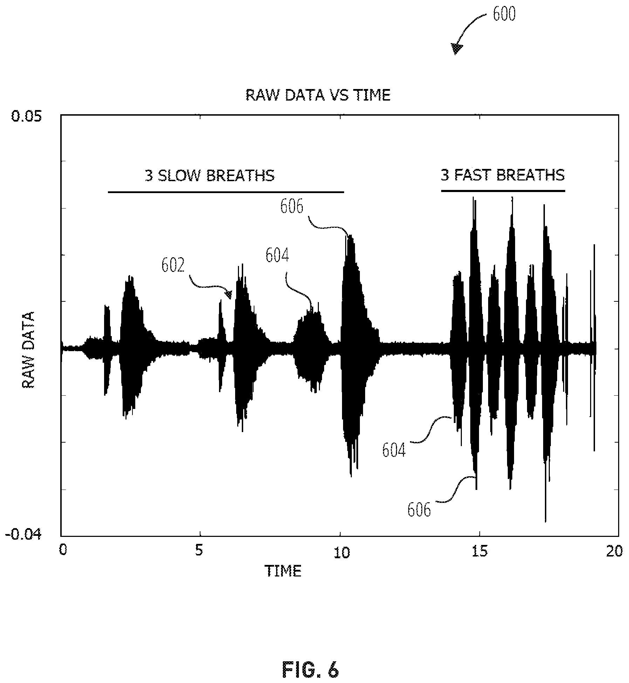

FIG. 6 is a graph 600 of an example of a signal 602 received by the breath signal analysis module 410 when the audio processor 400 is operating in a breath analysis mode, either from noise suppressor 406 or accelerometer signal processor 412. The illustrated signal 602 shows three slow breaths followed by three fast breaths. Each breath can be seen to comprise an initial amplitude peak 604 resulting from the sound generated during an inhale, followed by a second amplitude peak 606 resulting from the sound generated during an exhale.

FIG. 7A is a graph showing an example audio frequency distribution of an inhale, while FIG. 7B shows the audio frequency distribution of a corresponding exhale. As can be seen from FIG. 7A, there is a relatively equal distribution of frequencies in the range from 100 Hz to 1 kHz for the inhale, while, as can be seen from FIG. 7B, there is a sharp decline in the distribution of frequencies from 100 Hz to 1 kHz for the exhale. Additionally, there is a strong peak between 80 and 100 Hz for the inhale, which is much greater than the peak between 30 and 60 kHz for an inhale. Using these differences, the breath signal analysis module 410 can distinguish between an inhale and an exhale by comparing the two frequency distributions or by comparing the peak magnitudes between an inhale and an exhale.

Using signal processing techniques, the breath signal analysis module 410 determines various parameters and metrics from the audio signal received from the beamformer 402 and/or the noise suppressor 406, including the duration between breaths (i.e. the period), the depth of breathing, frequency characteristics, hyperventilation, asthmatic breathing, and stressful breathing.

For example, the breath signal analysis module 410 can determine the period between breaths (and thus its reciprocal, the breath frequency, also known as the respiratory rate) by doing a time window average of the amplitude of the audio signal samples. When the average of the signal value peaks, this can be considered to be a reliable indicator of a repeatable position in the breathing cycle corresponding to a transition from exhale to inhale. Additionally, the peak between inhale and exhale can be distinguished from the peak at the transition between exhale and inhale because the former will be lower than the latter. Also, as discussed above, an inhale can also be distinguished from an inhale based on the difference in frequency composition between the two.

By observing the respiratory rate (typically expressed as number of breaths per minute) or the period between breaths, as well as the frequency characteristics of the inhale and exhale, the breath signal analysis module 410 can make a number of determinations. For example, it can be determined that a user is breathing deeply by observing that a user's breath is slow and steady, for example with little variation in the period of consecutive breaths with a breath period of 8 seconds or more.

Similarly, if the person is breathing rapidly with a varying breath period, the breath signal analysis module 410 may determine that the person is hyperventilating. Additionally, if the audio frequency distribution includes stronger high frequency components, the breath signal analysis module 410 may determine that asthmatic breathing is taking place.

The determinations made by the breath signal analysis module 410 may be done by comparing average/reference respiratory rates and frequency profiles, optionally filtered by demographic characteristics such as age and gender, with the observed respiratory rates and frequency profiles. The determinations made by the breath signal analysis module 410 may also be done by comparing historical respiratory rates and frequency profiles for a particular user, based on previously observed breath data for that particular user. This data or characteristics derived therefrom may be stored on the application servers 3 12 associated with a particular user's account.

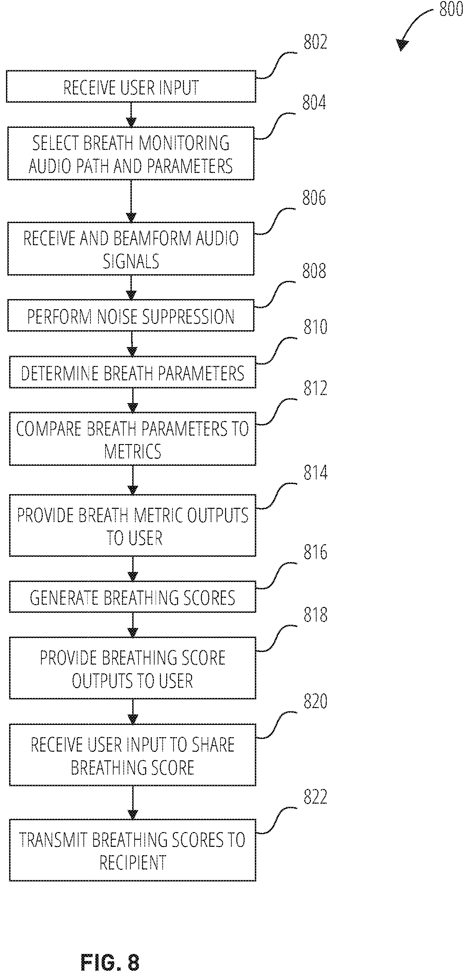

FIG. 8 is an example flow diagram of a breath monitoring process 800 using the head-wearable apparatus 100 from FIG. 1 according to various aspects of the disclosure. Although the flowchart may describe the operations as a sequential process, many of the operations can be performed in parallel or concurrently. In addition, the order of the operations may be re-arranged. A process is terminated when its operations are completed. A process may correspond to a method, a procedure, etc. The steps of method may be performed in whole or in part, may be performed in conjunction with some or all of the steps in other methods, and may be performed by any number of different systems, such as any of the systems described herein. The process 800 may also be performed by a processor included in head-wearable apparatus 100 in FIG. 1 or by a processor included in a client device 302.

The process 800 starts at operation 802 with the head-wearable apparatus 100 or the client device 302 receiving user input to begin operating in a breath monitoring and analysis mode. User input may be provided for example by button presses or touch input on the head-wearable apparatus 100 or the client device 302, voice commands, and so forth. In one embodiment, the breath monitoring mode is provided as an option in the messaging client 304 or an application 306 running on client device 302. For purposes of clarity only, process 800 will be described with respect to breath monitoring and analysis being implemented in the messaging client 304 on the client device 302 in conjunction with the head-wearable apparatus 100.

Upon receipt of the user input, in operation 804 the messaging client 304 enables the audio processor 400 (if it is not already enabled) and selects the audio processing path in the audio processor 400 to the path including the breath signal analysis module 410 in FIG. 4. The beamformer 402 is set so that gains and delays on the signals captured by the microphones 502a to 502e provide audio beamforming directed at the nose and mouth of a wearer of the head-wearable apparatus 100. The messaging client may also select or enable additional or different microphones compared to the monaural or binaural modes, such as the microphone 502e/110_5.

Audio signals are then received from the microphones 502a to 502e and processed by beamformer 402 at operation 806 to generate an output signal in which sounds emanating from a user's mouth and nose are enhanced. Any noise suppression provided by the audio processor 400 is then performed on the signal by the noise suppressor 406 and the resulting signal is passed to the breath signal analysis module 410.

Noise suppression (if any) is then performed in operation 808 by the noise suppressor 406 on the beamformed audio signal received from the beamformer 402 in operation 806.

The breath signal analysis module 410 then determines or generates breath parameters or characteristics from the resulting signal at operation 810. As discussed above, the breath parameters characterize the user's breathing and may include a respiration rate or period, peak amplitude values to identify exhalation vs inhalation, the frequency characteristics of the inhalation and exhalation, the variation in respiration rate or period etc. Breath parameters may also be derived from a signal received from the accelerometer signal processor 412.

At operation 812, the breath parameters determined in operation 810 are compared to various metrics. The metrics may for example be a desired respiration rate, undesirable respiration rates (e.g. that the respiration rate is above a certain threshold), frequency characteristics (e.g. the presence of high frequency components indicating wheezing), or any of the characteristics described above. The comparison may be to standard/average/reference metrics, which may or may not be refined by age, gender, and so forth.

Additionally, the comparison may be to historical information captured for the specific user, which may be beneficial to provide a baseline for the comparison. For example, if the frequency characteristics of a user's breathing has changed from its historical frequency characteristics, this may be an indication of an issue that should be brought to the user's attention.

At operation 814, the messaging client 304 provides breath-related output to the client device 302. This could be for example one or more basic parameters such as respiration rate, or parameters or notifications derived therefrom or as a result of any comparisons performed in operation 814. For example, if the frequency characteristics of the user's breathing is different from a general or historical metric, the messaging client may provide an appropriate notification, such as on the display screen of the client device 302, via audible prompt or other method. In another example, the messaging client 304 may provide audible or visual prompts relating to the user's breathing. For example, if the breath signal analysis module 410 or messaging client 304 determines the person is breathing rapidly, a visual or audible prompt to breathe more slowly and deeply may be provided. If the breath signal analysis module 410 or messaging client 304 determines that the person is breathing appropriately slowly and deeply, encouraging prompts may be provided to give positive feedback to the user.

The output provided by the messaging client 304 to the user may also include a visual or audible output that varies at varies at a desired respiration rate. For example, an expanding and contracting visualization may be provided or a varying audio output may be provided. Alternatively verbal breathing instructions may be provided at a desired cadence.

The messaging client 304 may also generate breathing scores from the breath parameters and their variation throughout a session in operation 816. For example, a dynamic accuracy score may be determined based on how closely the user's respiration matches a prompted or desired respiration rate. A cumulative score may be generated based on a combination of the dynamic accuracy score and the total amount of time that the messaging client 304 determines the user has spent doing breathing exercises or has achieved a certain level or target. For example, the messaging client might have a threshold difference between a desired or prompted respiration rate, and when a magnitude of the difference between the user's respiration rate and the prompted or desired rate is below the threshold, the messaging client 304 may the amount of time spent under the threshold as a relaxed breathing period and generate a breathing score based thereon. On the other hand, the amount of time a user spends above the threshold may be logged as a stressed breathing period. The amount of time spent by a user in such zones can be reported by the messaging client 304 to the user based on daily, weekly, monthly, yearly totals.

At operation 818, the messaging client 304 may provide breathing score outputs to the user, including the dynamic accuracy score and daily, weekly, monthly, yearly totals, and so forth. on the display of the client device 302. This may be in response to messaging client 304 receiving user selection of a "View Scores" option in a user interface of the messaging client 304 and the breathing scores may be displayed in textual or graphical formats on the display of the client device 302.

The display of the breathing scores may include a "Share Scores" option, which when selected by the user in operation 820, creates a message in an existing or new conversation (e.g., a chat session) in the user interface of the messaging client 304, with one or more of the breathing scores selected by the user for sharing. The conversation may be with a recipient or a group of recipients selected from a list of friends of the user of a client device 302. In some cases, the group of friends are friends of the user who also use the breath monitoring functionality that is included with or accessed via messaging client 304 or an external resource.

Upon selection of the recipients, the messaging client 304 (or external resource) transmits the breathing scores to the selected recipients in operation 822 in a message transmitted over the network 310. The message may include any of the breathing scores, for example a daily score of relaxed or stressed breathing periods.

It will be appreciated that the process 800 illustrated in FIG. 8 is, for many of the operations shown, a continuous process. For example, unless the user provides input to terminate the breath monitoring method or the path of the audio is diverted to the monaural audio processor 404 by the client device 302 due to an incoming call or to the binaural audio processor 408 to, for example, provide audio for video capture, operation 806 to operation 814 will continue. Additional operations may then occur automatically or upon user input.

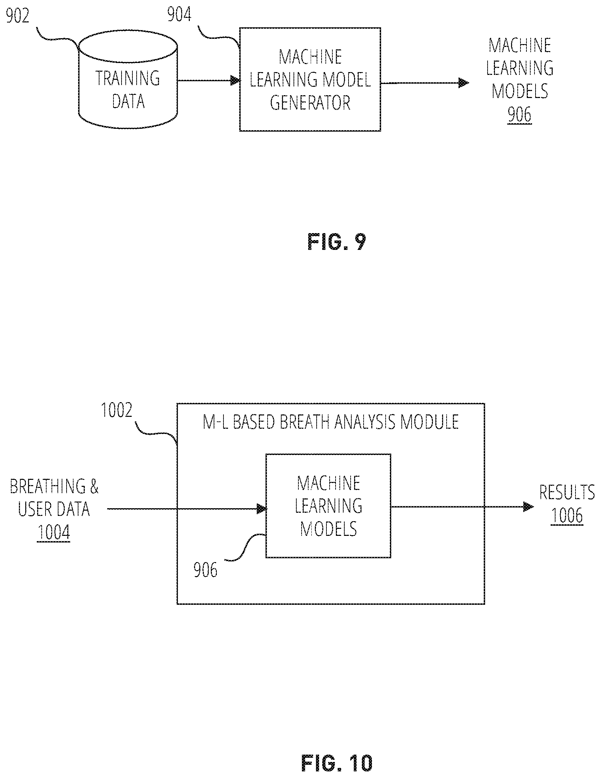

FIG. 9 is a diagrammatic representation illustrating the generation of machine learning models for use in breathing analysis, in accordance with some examples. As will be described in more detail below, in some examples a machine learning model can be used in place of or in addition to the breath signal analysis module 410 described above.

In one or more embodiments, the machine learning models 906 are implemented by one or more software modules (e.g., running on the messaging server system 110). In another example, the machine learning models 906 are implemented by one or more software modules implemented by custom hardware (e.g., one or more coprocessors). Not all of the depicted components may be used in all implementations, however, and one or more embodiments may include additional or different components than those shown in the figure. Variations in the arrangement and type of the components may be made without departing from the spirit or scope of the claims as set forth herein. Additional components, different components, or fewer components may be provided.

As shown in FIG. 9, a machine learning model generator 904 receives breathing analysis training data 902 as input. The training data 902 will, for example include different sets of breath-related data such as that generated by breath signal analysis module 410, which may include the duration between breaths (i.e. the period), the depth of breathing, frequency characteristics, and so forth. The breath training data will also include information about the nature of the breathing and any associated emotional or physical state, for example if the person was hyperventilating, breathing asthmatically, was feeling stressed or angry, and so forth. Demographic information will also typically be included, such as the person's age, gender, body type, the presence of acute or chronic medical problems related to breathing, fitness level and so forth. Based on the training data, the machine learning model generator 904 generates and trains the machine learning models 906 to recognize the nature of the breathing and any associated emotional states based on breath characteristics and demographic information of a user of the head-wearable apparatus 100 from which breath signal 602 are being captured.

The machine learning model generator 904 is configured to generate the machine learning models 906 using the training data 902as input. For example, the machine learning model generator 904 is configured to train, test and/or otherwise tune the machine learning models 906 based on the breathing data sets included in the training data 902. Examples of algorithms that may be employed by the machine learning model generator 904or training and/or testing the machine learning model generator 904include, but are not limited to, linear regression, boosted trees, multi-layer perceptron and/or random forest algorithms.

FIG. 10 is a diagrammatic representation illustrating the use of the machine learning models generated as described in FIG. 9, to perform breathing analysis. In FIG. 10, captured breathing & user data 1004 are provided as input to the M-L Based breath analysis module 1002, which includes one or more of the machine learning models 906. As described above, the captured data in some examples comprises data generated by breath signal analysis module 410, which may include the duration between breaths (i.e. the period), the depth of breathing, frequency characteristics, and so forth. User data, including demographic information about the user, such as the person's age, gender, body type, the presence of acute or chronic medical problems related to breathing, fitness level and so forth are provided from a user profile that is maintained in the head-wearable apparatus 100, the client device 302 or in the messaging server system 308. Based on the breathing & user data 1004, the breath signal M-L Based breath analysis module 1002 provides results 1006 indicating the nature of the person's breathing as well as any associated emotional state.

As before, the messaging client 304 can provide the breath-related analysis results 1006 to the user, via a notification on or by the client device 302, including the assessment of the person's breathing as well as any associated emotional state, as well as more basic parameters such as respiration rate, or parameters or notifications derived therefrom or as a result of any comparisons performed between current and previous data. The notification provided by the messaging client 304 may solicit feedback from the user. The messaging client 304 or messaging server system 308 may provide the user's feedback and breath data as positive and/or negative feedback to the machine learning model generator 904, for continuous/updated training of the machine learning models 906. In one or more embodiments, the positive and/or negative feedback may be provided across multiple users of the messaging server system 110, such that the machine learning model machine learning models 906 is continuously trained in a crowd-sourced manner. The users' modifications and positive/negative feedback is provided to the messaging server system 110 in an anonymous, encrypted manner in order to preserve user privacy.

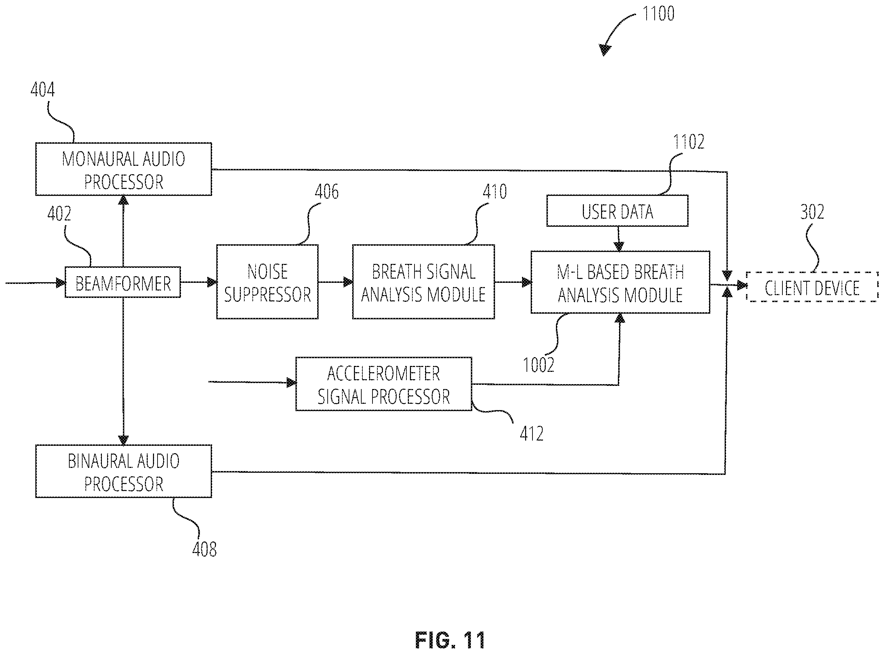

FIG. 11 illustrates a block diagram of an audio processor 1100 for use with head-wearable apparatus 100 according to some examples. The audio processor 1100 may be implemented in the head-wearable apparatus 100 or in an associated computing device such as client device 302. One embodiment of a client device 302 is machine 1300 discussed in more detail below with reference to FIG. 13. As with the audio processor 400 of FIG. 4, the audio processor 1100 in one example includes a beamformer 402, a monaural audio processor 404, a binaural audio processor 408, and a noise suppressor 406, which function as described above with reference to FIG. 4.

The audio processor 1100 includes an M-L Based breath analysis module 1002 that functions as described above with reference to FIG. 10. The M-L Based breath analysis module 1002 receives data generated by breath signal analysis module 410, which may include the duration between breaths (i.e. the period), the depth of breathing, frequency characteristics, and so forth. User data 1102, including demographic information about the user, such as the person's age, gender, body type, the presence of acute or chronic medical problems related to breathing, fitness level and so forth are provided from a user profile that is maintained in the head-wearable apparatus 100, the client device 302 or in the messaging server system 308.

As with the audio processor 400, the audio processor 1100 is able to switch between different modes depending on user input or automatically by the user device, depending on context. It will also be appreciated that while three signal paths are shown in FIG. 11 for processing audio from the microphones, the audio processor 1100 may also be implemented as a single adaptive audio path to provide the required processing. Additionally, more than one beamformer 402 may be included in the audio processor 1100, to provide enhancement of signals at more than one spatial location, such as in a binaural implementation.

The outputs of monaural audio processor 404, M-L Based breath analysis module 1002, and binaural audio processor 408 are provided to client device 302 (if audio processor 1 100 is not embodied in client device 302) over data link 328 for provision to the messaging client 304 or the application 306, or are provided to the messaging client 304 or application 306 if the audio processor 1100 is embodied in the client device 302. It will also be appreciated that various functions or modules of the audio processor 1100 may be distributed between the head-wearable apparatus 100 and the client device 302. The messaging client 304 or messaging server system 308 may also provide the user's feedback and breath data as positive and/or negative feedback to the machine learning model generator 904, for continuous/updated training of the machine learning models 906 as described above.

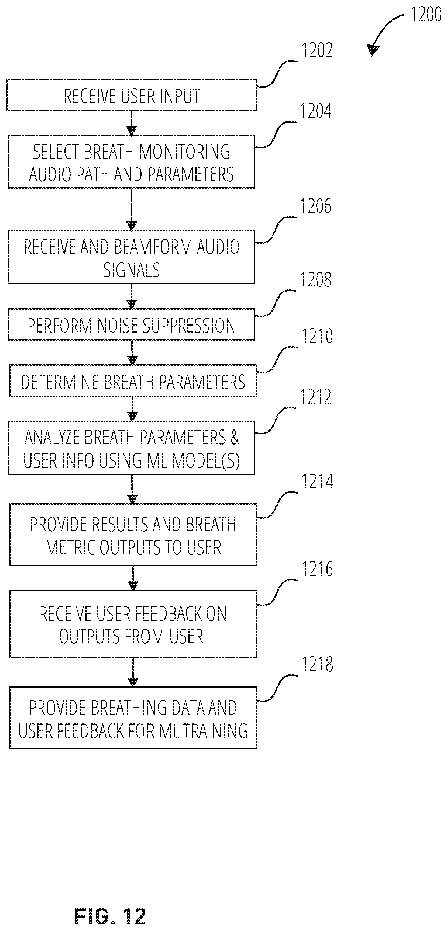

FIG. 12 is an example flow diagram of a breath monitoring process 1200 using the head-wearable apparatus 100 from FIG. 1 according to various aspects of the disclosure. Although the flowchart may describe the operations as a sequential process, many of the operations can be performed in parallel or concurrently. In addition, the order of the operations may be re-arranged. A process is terminated when its operations are completed. A process may correspond to a method, a procedure, etc. The steps of method may be performed in whole or in part, may be performed in conjunction with some or all of the steps in other methods, and may be performed by any number of different systems, such as any of the systems described herein. The process 1200 may also be performed by a processor included in head-wearable apparatus 100 in FIG. 1 or by a processor included in a client device 302.

The process 1200 starts at operation 1202 with the head-wearable apparatus 100 or the client device 302 receiving user input to begin operating in a breath monitoring and analysis mode. User input may be provided for example by button presses or touch input on the head-wearable apparatus 100 or the client device 302, voice commands, and so forth. In one embodiment, the breath monitoring mode is provided as an option in the messaging client 304 or an application 306 running on client device 302. For purposes of clarity only, process 1200 will be described with respect to breath monitoring and analysis being implemented in the messaging client 304 on the client device 302 in conjunction with the head-wearable apparatus 100.

Upon receipt of the user input, in operation 1204 the messaging client 304 enables the audio processor 1100 (if it is not already enabled) and selects the audio processing path in the audio processor 400 to the path including the breath signal analysis module 410 in FIG. 11. The beamformer 402 is set so that gains and delays on the signals captured by the microphones 502a to 502e provide audio beamforming directed at the nose and mouth of a wearer of the head-wearable apparatus 100. The messaging client may also select or enable additional or different microphones compared to the monaural or binaural modes, such as the microphone 502e/110_5.

Audio signals are then received from the microphones 502a to 502e and processed by beamformer 402 at operation 1206 to generate an output signal in which sounds emanating from a user's mouth and nose are enhanced. Any noise suppression provided by the audio processor 400 is then performed on the signal by the noise suppressor 406 and the resulting signal is passed to the breath signal analysis module 410.

Noise suppression (if any) is then performed in operation 1208 by the noise suppressor 406 on the beamformed audio signal received from the beamformer 402 in operation 1206.

The breath signal analysis module 410 then determines or generates breath parameters or characteristics from the resulting signal at operation 1210. As discussed above, the breath parameters characterize the user's breathing and may include a respiration rate or period, peak amplitude values to identify exhalation vs inhalation, the frequency characteristics of the inhalation and exhalation, the variation in respiration rate or period etc. Breath parameters may also be derived from a signal received from the accelerometer signal processor 412.

At operation 1212, the breath parameters determined in operation 1210 are, together with User data 1102 such as demographic information are processed by the M-L Based breath analysis module 1002 using the machine learning models 906, to determine an assessment of the nature of the breathing and any associated emotional or physical state, for example if the person was hyperventilating, breathing asthmatically, was feeling stressed or angry, and so forth.

As for the process 800 of FIG. 8, various breathing metrics may also be determined in 1212. The metrics may for example be a desired respiration rate, undesirable respiration rates (e.g. that the respiration rate is above a certain threshold), frequency characteristics (e.g. the presence of high frequency components indicating wheezing), or any of the characteristics described above. A comparison between actual and desired breathing metrics may be to standard/average/reference metrics, which may or may not be refined by age, gender, and so forth. Further, the comparison may be to historical information captured for the specific user, which may be beneficial to provide a baseline for the comparison. For example, if the frequency characteristics of a user's breathing has changed from its historical frequency characteristics, this may be an indication of an issue that should be brought to the user's attention.

At operation 1214, the messaging client 304 provides breath-related output to the client device 302. This is the nature of the breathing or any associated emotional or physical state determined by the M-L Based breath analysis module 1002. Additionally, as described above with reference to FIG. 8,one or more basic parameters such as respiration rate, or parameters or notifications derived therefrom or as a result of any comparisons performed in operation 1212 may also be provided, in conjunction with the machine-learning-derived output. Furthermore, historical information may also be provided.

In operation 1216, the messaging client 304 receives user feedback, in particular on the nature of the breathing and any associated emotional or physical state as determined by the M-L Based breath analysis module 1002 using the machine learning models 906. For example, the messaging client may display a prompt that says: "It looks like you're [emotional state]. Is that correct?" If the messaging client receives a positive response, breathing exercises or other tips can be provided to assist with the emotional state. If the response is negative, the messaging client 304 provides prompts to elicit feedback from the user on the nature of their breathing, their emotional state or their physical state. For example, "Are you having or are you (select one or more of the following): a panic attack, an asthma attack, angry, upset, excited..." and so forth.

In operation 1218, the user's feedback and breath data are provided as positive and/or negative feedback to the machine learning model generator 904, for continuous/updated training of the machine learning models 906

It will be appreciated that the process 1200 illustrated in FIG. 12 is, for many of the operations shown, a continuous process. For example, unless the user provides input to terminate the breath monitoring method or the path of the audio is diverted to the monaural audio processor 404 by the client device 302 due to an incoming call or to the binaural audio processor 408 to, for example, provide audio for video capture, operation 1206 to operation 1214 will continue. Additional operations may then occur automatically or upon user input.

FIG. 13 is a diagrammatic representation of the machine 1300 within which instructions 1310 (e.g., software, a program, an application, an applet, an app, or other executable code) for causing the machine 1300 to perform any one or more of the methodologies discussed herein may be executed. For example, the instructions 1310 may cause the machine 1300 to execute any one or more of the methods described herein. The instructions 1310 transform the general, non-programmed machine 1300 into a particular machine 1300 programmed to carry out the described and illustrated functions in the manner described. The machine 1300 may operate as a standalone device or may be coupled (e.g., networked) to other machines. In a networked deployment, the machine 1300 may operate in the capacity of a server machine or a client machine in a server-client network environment, or as a peer machine in a peer-to-peer (or distributed) network environment. The machine 1300 may comprise, but not be limited to, a server computer, a client computer, a personal computer (PC), a tablet computer, a laptop computer, a netbook, a set-top box (STB), a personal digital assistant (PDA), an entertainment media system, a cellular telephone, a smartphone, a mobile device, a wearable device (e.g., a smartwatch), a smart home device (e.g., a smart appliance), other smart devices, a web appliance, a network router, a network switch, a network bridge, or any machine capable of executing the instructions 1310, sequentially or otherwise, that specify actions to be taken by the machine 1300. Further, while only a single machine 1300 is illustrated, the term "machine" shall also be taken to include a collection of machines that individually or jointly execute the instructions 1310 to perform any one or more of the methodologies discussed herein. The machine 1300, for example, may comprise the client device 302 or any one of a number of server devices forming part of the messaging server system 308. In some examples, the machine 1300 may also comprise both client and server systems, with certain operations of a particular method or algorithm being performed on the server-side and with certain operations of the particular method or algorithm being performed on the client-side.

The machine 1300 may include processors 1304, memory 1306, and input/output I/O components 1302, which may be configured to communicate with each other via a bus 1340. In an example, the processors 1304 (e.g., a Central Processing Unit (CPU), a Reduced Instruction Set Computing (RISC) Processor, a Complex Instruction Set Computing (CISC) Processor, a Graphics Processing Unit (GPU), a Digital Signal Processor (DSP), an Application Specific Integrated Circuit (ASIC), a Radio-Frequency Integrated Circuit (RFIC), another processor, or any suitable combination thereof) may include, for example, a processor 1308 and a processor 13 12 that execute the instructions 13 10. The term "processor" is intended to include multi-core processors that may comprise two or more independent processors (sometimes referred to as "cores") that may execute instructions contemporaneously. Although FIG. 13 shows multiple processors 1304, the machine 1300 may include a single processor with a single-core, a single processor with multiple cores (e.g., a multi-core processor), multiple processors with a single core, multiple processors with multiples cores, or any combination thereof.

The memory 1306 includes a main memory 1314, a static memory 1316, and a storage unit 1318, both accessible to the processors 1304 via the bus 1340. The main memory 1306, the static memory 1316, and storage unit 1318 store the instructions 1310 embodying any one or more of the methodologies or functions described herein. The instructions 1310 may also reside, completely or partially, within the main memory 1314, within the static memory 1316, within machine-readable medium 1320 within the storage unit 1318, within at least one of the processors 1304 (e.g., within the Processor's cache memory), or any suitable combination thereof, during execution thereof by the machine 1300.

The I/O components 1302 may include a wide variety of components to receive input, provide output, produce output, transmit information, exchange information, capture measurements, and so on. The specific I/O components 1302 that are included in a particular machine will depend on the type of machine. For example, portable machines such as mobile phones may include a touch input device or other such input mechanisms, while a headless server machine will likely not include such a touch input device. It will be appreciated that the I/O components 1302 may include many other components that are not shown in FIG. 13. In various examples, the I/O components 1302 may include user output components 1326 and user input components 1328. The user output components 1326 may include visual components (e.g., a display such as a plasma display panel (PDP), a light-emitting diode (LED) display, a liquid crystal display (LCD), a projector, or a cathode ray tube (CRT)), acoustic components (e.g., speakers), haptic components (e.g., a vibratory motor, resistance mechanisms), other signal generators, and so forth. The user input components 1328 may include alphanumeric input components (e.g., a keyboard, a touch screen configured to receive alphanumeric input, a photo-optical keyboard, or other alphanumeric input components), point-based input components (e.g., a mouse, a touchpad, a trackball, a joystick, a motion sensor, or another pointing instrument), tactile input components (e.g., a physical button, a touch screen that provides location and force of touches or touch gestures, or other tactile input components), audio input components (e.g., a microphone), and the like.

In further examples, the I/O components 1302 may include biometric components 1330, motion components 1332, environmental components 1334, or position components 1336, among a wide array of other components. For example, the biometric components 1330 include components to detect expressions (e.g., hand expressions, facial expressions, vocal expressions, body gestures, or eye-tracking), measure biosignals (e.g., blood pressure, heart rate, body temperature, perspiration, or brain waves), identify a person (e.g., voice identification, retinal identification, facial identification, fingerprint identification, or electroencephalogram-based identification), and the like. The motion components 1332 include acceleration sensor components (e.g., accelerometer), gravitation sensor components, rotation sensor components (e.g., gyroscope).

The environmental components 1334 include, for example, one or cameras (with still image/photograph and video capabilities), illumination sensor components (e.g., photometer), temperature sensor components (e.g., one or more thermometers that detect ambient temperature), humidity sensor components, pressure sensor components (e.g., barometer), acoustic sensor components (e.g., one or more microphones that detect background noise), proximity sensor components (e.g., infrared sensors that detect nearby objects), gas sensors (e.g., gas detection sensors to detection concentrations of hazardous gases for safety or to measure pollutants in the atmosphere), or other components that may provide indications, measurements, or signals corresponding to a surrounding physical environment.

With respect to cameras, the client device 302 may have a camera system comprising, for example, front cameras on a front surface of the client device 302 and rear cameras on a rear surface of the client device 302. The front cameras may, for example, be used to capture still images and video of a user of the client device 302 (e.g., "selfies"), which may then be augmented with augmentation data (e.g., filters) described above. The rear cameras may, for example, be used to capture still images and videos in a more traditional camera mode, with these images similarly being augmented with augmentation data. In addition to front and rear cameras, the client device 302 may also include a 360° camera for capturing 360° photographs and videos.

Further, the camera system of a client device 302 may include dual rear cameras (e.g., a primary camera as well as a depth-sensing camera), or even triple, quad or penta rear camera configurations on the front and rear sides of the client device 302. These multiple cameras systems may include a wide camera, an ultra-wide camera, a telephoto camera, a macro camera and a depth sensor, for example.

The position components 1336 include location sensor components (e.g., a GPS receiver component), altitude sensor components (e.g., altimeters or barometers that detect air pressure from which altitude may be derived), orientation sensor components (e.g., magnetometers), and the like.

Communication may be implemented using a wide variety of technologies. The I/O components 1302 further include communication components 1338 operable to couple the machine 1300 to a network 1322 or devices 1324 via respective coupling or connections. For example, the communication components 1338 may include a network interface Component or another suitable device to interface with the network 1322. In further examples, the communication components 1338 may include wired communication components, wireless communication components, cellular communication components, Near Field Communication (NFC) components, Bluetooth^{®} components (e.g., Bluetooth^{®} Low Energy), Wi-Fi^{®} components, and other communication components to provide communication via other modalities. The devices 1324 may be another machine or any of a wide variety of peripheral devices (e.g., a peripheral device coupled via a USB).

Moreover, the communication components 1338 may detect identifiers or include components operable to detect identifiers. For example, the communication components 1338 may include Radio Frequency Identification (RFID) tag reader components, NFC smart tag detection components, optical reader components (e.g., an optical sensor to detect one-dimensional bar codes such as Universal Product Code (UPC) bar code, multidimensional bar codes such as Quick Response (QR) code, Aztec code, Data Matrix, Dataglyph, MaxiCode, PDF417, Ultra Code, UCC RSS-2D bar code, and other optical codes), or acoustic detection components (e.g., microphones to identify tagged audio signals). In addition, a variety of information may be derived via the communication components 1338, such as location via Internet Protocol (IP) geolocation, location via Wi-Fi^{®} signal triangulation, location via detecting an NFC beacon signal that may indicate a particular location, and so forth.

The various memories (e.g., main memory 1314, static memory 1316, and memory of the processors 1304) and storage unit 1318 may store one or more sets of instructions and data structures (e.g., software) embodying or used by any one or more of the methodologies or functions described herein. These instructions (e.g., the instructions 13 10), when executed by processors 1304, cause various operations to implement the disclosed examples.

The instructions 1310 may be transmitted or received over the network 1322, using a transmission medium, via a network interface device (e.g., a network interface component included in the communication components 1338) and using any one of several well-known transfer protocols (e.g., hypertext transfer protocol (HTTP)). Similarly, the instructions 1310 may be transmitted or received using a transmission medium via a coupling (e.g., a peer-to-peer coupling) to the devices 1324.

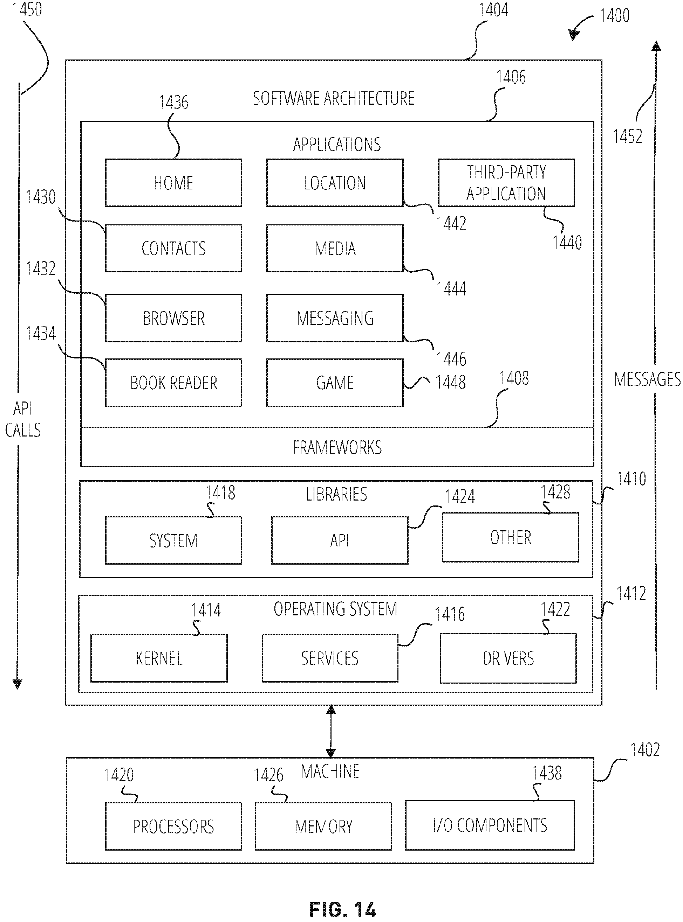

FIG. 14 is a block diagram 1400 illustrating a software architecture 1404, which can be installed on any one or more of the devices described herein. The software architecture 1404 is supported by hardware such as a machine 1402 that includes processors 1420, memory 1426, and I/O components 1438. In this example, the software architecture 1404 can be conceptualized as a stack of layers, where each layer provides a particular functionality. The software architecture 1404 includes layers such as an operating system 1412, libraries 1410, frameworks 1408, and applications 1406. Operationally, the applications 1406 invoke API calls 1450 through the software stack and receive messages 1452 in response to the API calls 1450.

The operating system 1412 manages hardware resources and provides common services. The operating system 1412 includes, for example, a kernel 1414, services 1416, and drivers 1422. The kernel 1414 acts as an abstraction layer between the hardware and the other software layers. For example, the kernel 1414 provides memory management, processor management (e.g., scheduling), component management, networking, and security settings, among other functionality. The services 1416 can provide other common services for the other software layers. The drivers 1422 are responsible for controlling or interfacing with the underlying hardware. For instance, the drivers 1422 can include display drivers, camera drivers, BLUETOOTH^{®} or BLUETOOTH^{®} Low Energy drivers, flash memory drivers, serial communication drivers (e.g., USB drivers), WI-FI^{®} drivers, audio drivers, power management drivers, and so forth.

The libraries 1410 provide a common low-level infrastructure used by the applications 1406. The libraries 1410 can include system libraries 1418 (e.g., C standard library) that provide functions such as memory allocation functions, string manipulation functions, mathematic functions, and the like. In addition, the libraries 1410 can include API libraries 1424 such as media libraries (e.g., libraries to support presentation and manipulation of various media formats such as Moving Picture Experts Group-4 (MPEG4), Advanced Video Coding (H.264 or AVC), Moving Picture Experts Group Layer-3 (MP3), Advanced Audio Coding (AAC), Adaptive Multi-Rate (AMR) audio codec, Joint Photographic Experts Group (JPEG or JPG), or Portable Network Graphics (PNG)), graphics libraries (e.g., an OpenGL framework used to render in two dimensions (2D) and three dimensions (3D) in a graphic content on a display), database libraries (e.g., SQLite to provide various relational database functions), web libraries (e.g., WebKit to provide web browsing functionality), and the like. The libraries 1410 can also include a wide variety of other libraries 1428 to provide many other APIs to the applications 1406.

The frameworks 1408 provide a common high-level infrastructure that is used by the applications 1406. For example, the frameworks 1408 provide various graphical user interface (GUI) functions, high-level resource management, and high-level location services. The frameworks 1408 can provide a broad spectrum of other APIs that can be used by the applications 1406, some of which may be specific to a particular operating system or platform.

In an example, the applications 1406 may include a home application 1436, a contacts application 1430, a browser application 1432, a book reader application 1434, a location application 1442, a media application 1444, a messaging application 1446, a game application 1448, and a broad assortment of other applications such as a third-party application 1440. The applications 1406 are programs that execute functions defined in the programs. Various programming languages can be employed to create one or more of the applications 1406, structured in a variety of manners, such as object-oriented programming languages (e.g., Objective-C, Java, or C++) or procedural programming languages (e.g., C or assembly language). In a specific example, the third-party application 1440 (e.g., an application developed using the ANDROID^{™} or IOS^{™} software development kit (SDK) by an entity other than the vendor of the particular platform) may be mobile software running on a mobile operating system such as IOS^{™}, ANDROID^{™}, WINDOWS^{®} Phone, or another mobile operating system. In this example, the third-party application 1440 can invoke the API calls 1450 provided by the operating system 1412 to facilitate functionality described herein.

Where a phrase similar to "at least one of A, B, or C," "at least one of A, B, and C," "one or more A, B, or C," or "one or more of A, B, and C" is used, it is intended that the phrase be interpreted to mean that A alone may be present in an embodiment, B alone may be present in an embodiment, C alone may be present in an embodiment, or that any combination of the elements A, B and C may be present in a single embodiment; for example, A and B, A and C, B and C, or A and B and C.

### GLOSSARY

"Carrier signal" refers to any intangible medium that is capable of storing, encoding, or carrying instructions for execution by the machine, and includes digital or analog communications signals or other intangible media to facilitate communication of such instructions. Instructions may be transmitted or received over a network using a transmission medium via a network interface device.

"Client device" refers to any machine that interfaces to a communications network to obtain resources from one or more server systems or other client devices. A client device may be, but is not limited to, a mobile phone, desktop computer, laptop, portable digital assistants (PDAs), smartphones, tablets, ultra-books, netbooks, laptops, multi-processor systems, microprocessor-based or programmable consumer electronics, game consoles, set-top boxes, or any other communication device that a user may use to access a network.

"Communication network" refers to one or more portions of a network that may be an ad hoc network, an intranet, an extranet, a virtual private network (VPN), a local area network (LAN), a wireless LAN (WLAN), a wide area network (WAN), a wireless WAN (WWAN), a metropolitan area network (MAN), the Internet, a portion of the Internet, a portion of the Public Switched Telephone Network (PSTN), a plain old telephone service (POTS) network, a cellular telephone network, a wireless network, a Wi-Fi^{®} network, another type of network, or a combination of two or more such networks. For example, a network or a portion of a network may include a wireless or cellular network and the coupling may be a Code Division Multiple Access (CDMA) connection, a Global System for Mobile communications (GSM) connection, or other types of cellular or wireless coupling. In this example, the coupling may implement any of a variety of types of data transfer technology, such as Single Carrier Radio Transmission Technology (1xRTT), Evolution-Data Optimized (EVDO) technology, General Packet Radio Service (GPRS) technology, Enhanced Data rates for GSM Evolution (EDGE) technology, third Generation Partnership Project (3GPP) including 3G, fourth generation wireless (4G) networks, Universal Mobile Telecommunications System (UMTS), High Speed Packet Access (HSPA), Worldwide Interoperability for Microwave Access (WiMAX), Long Term Evolution (LTE) standard, others defined by various standard-setting organizations, other long-range protocols, or other data transfer technology.

"Component" refers to a device, physical entity, or logic having boundaries defined by function or subroutine calls, branch points, APIs, or other technologies that provide for the partitioning or modularization of particular processing or control functions. Components may be combined via their interfaces with other components to carry out a machine process. A component may be a packaged functional hardware unit designed for use with other components and a part of a program that usually performs a particular function of related functions. Components may constitute either software components (e.g., code embodied on a machine-readable medium) or hardware components. A "hardware component" is a tangible unit capable of performing certain operations and may be configured or arranged in a certain physical manner. In various examples, one or more computer systems (e.g., a standalone computer system, a client computer system, or a server computer system) or one or more hardware components of a computer system (e.g., a processor or a group of processors) may be configured by software (e.g., an application or application portion) as a hardware component that operates to perform certain operations as described herein. A hardware component may also be implemented mechanically, electronically, or any suitable combination thereof. For example, a hardware component may include dedicated circuitry or logic that is permanently configured to perform certain operations. A hardware component may be a special-purpose processor, such as a field-programmable gate array (FPGA) or an application specific integrated circuit (ASIC). A hardware component may also include programmable logic or circuitry that is temporarily configured by software to perform certain operations. For example, a hardware component may include software executed by a general-purpose processor or other programmable processor. Once configured by such software, hardware components become specific machines (or specific components of a machine) uniquely tailored to perform the configured functions and are no longer general-purpose processors. It will be appreciated that the decision to implement a hardware component mechanically, in dedicated and permanently configured circuitry, or in temporarily configured circuitry (e.g., configured by software), may be driven by cost and time considerations. Accordingly, the phrase "hardware component"(or "hardware-implemented component") should be understood to encompass a tangible entity, be that an entity that is physically constructed, permanently configured (e.g., hardwired), or temporarily configured (e.g., programmed) to operate in a certain manner or to perform certain operations described herein. Considering examples in which hardware components are temporarily configured (e.g., programmed), each of the hardware components need not be configured or instantiated at any one instance in time. For example, where a hardware component comprises a general-purpose processor configured by software to become a special-purpose processor, the general-purpose processor may be configured as respectively different special-purpose processors (e.g., comprising different hardware components) at different times. Software accordingly configures a particular processor or processors, for example, to constitute a particular hardware component at one instance of time and to constitute a different hardware component at a different instance of time. Hardware components can provide information to, and receive information from, other hardware components. Accordingly, the described hardware components may be regarded as being communicatively coupled. Where multiple hardware components exist contemporaneously, communications may be achieved through signal transmission (e.g., over appropriate circuits and buses) between or among two or more of the hardware components. In examples in which multiple hardware components are configured or instantiated at different times, communications between such hardware components may be achieved, for example, through the storage and retrieval of information in memory structures to which the multiple hardware components have access. For example, one hardware component may perform an operation and store the output of that operation in a memory device to which it is communicatively coupled. A further hardware component may then, at a later time, access the memory device to retrieve and process the stored output. Hardware components may also initiate communications with input or output devices, and can operate on a resource (e.g., a collection of information). The various operations of example methods described herein may be performed, at least partially, by one or more processors that are temporarily configured (e.g., by software) or permanently configured to perform the relevant operations. Whether temporarily or permanently configured, such processors may constitute processor-implemented components that operate to perform one or more operations or functions described herein. As used herein, "processor-implemented component" refers to a hardware component implemented using one or more processors. Similarly, the methods described herein may be at least partially processor-implemented, with a particular processor or processors being an example of hardware. For example, at least some of the operations of a method may be performed by one or more processors 1304 or processor-implemented components. Moreover, the one or more processors may also operate to support performance of the relevant operations in a "cloud computing" environment or as a "software as a service" (SaaS). For example, at least some of the operations may be performed by a group of computers (as examples of machines including processors), with these operations being accessible via a network (e.g., the Internet) and via one or more appropriate interfaces (e.g., an API). The performance of certain of the operations may be distributed among the processors, not only residing within a single machine, but deployed across a number of machines. In some examples, the processors or processor-implemented components may be located in a single geographic location (e.g., within a home environment, an office environment, or a server farm). In other examples, the processors or processor-implemented components may be distributed across a number of geographic locations.

"Computer-readable storage medium" refers to both machine-storage media and transmission media. Thus, the terms include both storage devices/media and carrier waves/modulated data signals. The terms "machine-readable medium," "computer-readable medium" and "device-readable medium" mean the same thing and may be used interchangeably in this disclosure.

"Ephemeral message" refers to a message that is accessible for a time-limited duration. An ephemeral message may be a text, an image, a video and the like. The access time for the ephemeral message may be set by the message sender. Alternatively, the access time may be a default setting or a setting specified by the recipient. Regardless of the setting technique, the message is transitory.

"Machine storage medium" refers to a single or multiple storage devices and media (e.g., a centralized or distributed database, and associated caches and servers) that store executable instructions, routines and data. The term shall accordingly be taken to include, but not be limited to, solid-state memories, and optical and magnetic media, including memory internal or external to processors. Specific examples of machine-storage media, computer-storage media and device-storage media include non-volatile memory, including by way of example semiconductor memory devices, e.g., erasable programmable read-only memory (EPROM), electrically erasable programmable read-only memory (EEPROM), FPGA, and flash memory devices; magnetic disks such as internal hard disks and removable disks; magneto-optical disks; and CD-ROM and DVD-ROM disks The terms "machine-storage medium," "device-storage medium," "computer-storage medium" mean the same thing and may be used interchangeably in this disclosure. The terms "machine-storage media," "computer-storage media," and "device-storage media" specifically exclude carrier waves, modulated data signals, and other such media, at least some of which are covered under the term "signal medium."

"Non-transitory computer-readable storage medium" refers to a tangible medium that is capable of storing, encoding, or carrying the instructions for execution by a machine.

"Signal medium" refers to any intangible medium that is capable of storing, encoding, or carrying the instructions for execution by a machine and includes digital or analog communications signals or other intangible media to facilitate communication of software or data. The term "signal medium" shall be taken to include any form of a modulated data signal, carrier wave, and so forth. The term "modulated data signal" means a signal that has one or more of its characteristics set or changed in such a matter as to encode information in the signal. The terms "transmission medium" and "signal medium" mean the same thing and may be used interchangeably in this disclosure.

## Claims

1. A method of monitoring the breathing of a user, performed by one or more processors, the method comprising:
receiving (1206) a plurality of audio signals from a plurality of microphones;
beamforming (1206) the plurality of audio signals into a beamformed audio signal;
determining (1210) observed breath-related parameters from the beamformed audio signal, wherein said step of determining breath-related parameters comprises
determining (1212) a nature of the breathing or a state of the user from the observed breath-related parameters using a trained machine learning model;
providing (1214) an output to the user of the nature of the breathing or a state of the user;
**characterised in that** the method further comprises:
requesting feedback from the user on the nature of the breathing or the state of the user;
receiving (1216) feedback from the user on the nature of the breathing or the state of the user; and
updating (1218) the trained machine learning model based on the feedback received from the user.

2. The method of claim 1 wherein the observed breath-related parameters include audio frequency distribution characteristics of an inhale or an exhale.

3. The method of claim 1 wherein the trained machine learning model determines the nature of the breathing or the state of the user at least in part by comparing the observed breath-related parameters with reference breath-related parameters.

4. The method of claim 3 wherein the reference breath-related parameters are historical breath-related parameters for the user.

5. The method of claim 3 wherein the observed breath-related parameters include audio frequency distribution characteristics of an inhale or an exhale.

6. The method of claim 3 wherein the reference breath-related parameters are demographic breath-related parameters based at least on age and gender.

7. The method of claim 3, further comprising:
determining from the determined nature of the breathing or a state of the user an accuracy score based on how closely an observed respiration of the user matches a prompted or desired respiration rate; and
providing an output to the user of the accuracy score.

8. A non-transitory machine-readable storage medium comprising instructions that, when executed by one or more processors of a machine, cause the machine to perform operations for monitoring the breathing of a user, the operations comprising:
receiving (1206) a plurality of audio signals from a plurality of microphones;
beamforming (1206) the plurality of audio signals into a beamformed audio signal;
determining (1210) observed breath-related parameters from the beamformed audio signal, wherein said step of determining breath-related parameters comprises
determining (1212) a nature of the breathing or a state of the user from the observed breath-related parameters using a trained machine learning model;
providing (1214) an output to the user of the nature of the breathing or a state of the user; **characterised in that** the operations comprise:
requesting feedback from the user on the nature of the breathing or the state of the user;
receiving (1216) feedback from the user on the nature of the breathing or the state of the user; and
updating (1218) the trained machine learning model based on the feedback received from the user.

9. The non-transitory machine-readable storage medium of claim 8, wherein the observed breath-related parameters include audio frequency distribution characteristics of an inhale or an exhale.

10. The non-transitory machine-readable storage medium of claim 8, wherein the trained machine learning model determines the nature of the breathing or the state of the user at least in part by comparing the observed breath-related parameters with reference breath-related parameters.

11. The non-transitory machine-readable storage medium of claim 10, wherein the operations further comprise:
determining from the determined nature of the breathing or a state of the user an accuracy score based on how closely an observed respiration rate of the user matches a prompted or desired respiration rate; and
providing an output to the user of the accuracy score.

12. The non-transitory machine-readable storage medium of claim 11 wherein the accuracy score is further based on an observed amount of time that the observed respiration rate is within a threshold value of the prompted or desired respiration rate.

13. A system comprising:
one or more processors (1304); and
one or more machine-readable mediums storing instructions that, when executed by the one or more processors (1304), cause the system to perform operations for monitoring the breathing of a user, the operations comprising:
receiving (1206) a plurality of audio signals from a plurality of microphones;
beamforming (1206) the plurality of audio signals into a beamformed audio signal;
determining (1210) observed breath-related parameters from the beamformed audio signal, wherein said step of determining breath-related parameters comprises
determining (1212) a nature of the breathing or a state of the user from the observed breath-related parameters using a trained machine learning model;
providing (1214) an output to the user of the nature of the breathing or a state of the user; **characterised in that** the operations comprise:
requesting feedback from the user on the nature of the breathing or the state of the user;
receiving (1216) feedback from the user on the nature of the breathing or the state of the user; and
updating (1218) the trained machine learning model based on the feedback received from the user.

14. The system of claim 13, wherein the observed breath-related parameters include audio frequency distribution characteristics of an inhale or an exhale.

15. The system of claim 13, wherein the operations further comprise:
deriving from the determined nature of the breathing or a state of the user a breathing score, the breathing score being based on a combination of how closely an observed respiration of the user matches a prompted or desired respiration rate and an observed amount of time that the observed respiration rate is within a threshold value of the prompted or desired respiration rate.

## Patentansprüche

1. Verfahren zum Überwachen der Atmung eines Benutzers, das von einem oder mehreren Prozessoren durchgeführt wird, wobei das Verfahren Folgendes umfasst:
Empfangen (1206) einer Vielzahl von Audiosignalen von einer Vielzahl von Mikrofonen;
Strahlausbilden (1206) der Vielzahl von Audiosignalen in ein strahlausgebildetes Audiosignal;
Bestimmen (1210) beobachteter atembezogener Parameter aus dem strahlausgebildeten Audiosignal, wobei der Schritt des Bestimmens atembezogener Parameter Folgendes umfasst:
Bestimmen (1212) einer Art der Atmung oder eines Zustands des Benutzers aus den beobachteten atembezogenen Parametern unter Verwendung eines trainierten maschinellen Lernmodells;
Bereitstellen (1214) einer Ausgabe der Art der Atmung oder eines Zustands des Benutzers an den Benutzer;
**dadurch gekennzeichnet, dass** das Verfahren ferner Folgendes umfasst:
Anfordern einer Rückmeldung des Benutzers über die Art der Atmung oder den Zustand des Benutzers;
Empfangen (1216) einer Rückmeldung von dem Benutzer über die Art der Atmung oder den Zustand des Benutzers; und
Aktualisieren (1218) des trainierten maschinellen Lernmodells auf Grundlage der von dem Benutzer empfangenen Rückmeldung.

2. Verfahren nach Anspruch 1, wobei die beobachteten atembezogenen Parameter die Audiofrequenzverteilungsmerkmale eines Einatmens oder eines Ausatmens enthalten.

3. Verfahren nach Anspruch 1, wobei das trainierte maschinelle Lernmodell die Art der Atmung oder den Zustand des Benutzers mindestens teilweise durch Vergleichen der beobachteten atembezogenen Parameter mit atembezogenen Referenzparametern bestimmt.

4. Verfahren nach Anspruch 3, wobei die atembezogenen Referenzparameter historische atembezogene Parameter für den Benutzer sind.

5. Verfahren nach Anspruch 3, wobei die beobachteten atembezogenen Parameter die Audiofrequenzverteilungsmerkmale eines Einatmens oder eines Ausatmens enthalten.

6. Verfahren nach Anspruch 3, wobei die atembezogenen Referenzparameter demographische atembezogene Parameter sind, die wenigstens auf Alter und Geschlecht basieren.

7. Verfahren nach Anspruch 3, das ferner Folgendes umfasst:
Bestimmen einer Genauigkeitsbewertungsziffer aus der bestimmten Art der Atmung oder einem Zustand des Benutzers auf Grundlage darauf, wie genau eine beobachtete Respiration des Benutzers mit einer angeforderten oder gewünschten Respirationsrate übereinstimmt; und
Bereitstellen einer Ausgabe der Genauigkeitsbewertungsziffer für den Benutzer.

8. Nicht transitorisches, maschinenlesbares Speichermedium, das Anweisungen umfasst, die, wenn sie von einem oder mehreren Prozessoren einer Maschine ausgeführt werden, die Maschine veranlassen, Vorgänge zum Überwachen der Atmung eines Benutzers durchzuführen, wobei die Vorgänge Folgendes umfassen:
Empfangen (1206) einer Vielzahl von Audiosignalen von einer Vielzahl von Mikrofonen;
Strahlausbilden (1206) der Vielzahl von Audiosignalen in ein strahlausgebildetes Audiosignal;
Bestimmen (1210) beobachteter atembezogener Parameter aus dem strahlausgebildeten Audiosignal, wobei der Schritt des Bestimmens atembezogener Parameter Folgendes umfasst:
Bestimmen (1212) einer Art der Atmung oder eines Zustands des Benutzers aus den beobachteten atembezogenen Parametern unter Verwendung eines trainierten maschinellen Lernmodells;
Bereitstellen (1214) einer Ausgabe der Art der Atmung oder eines Zustands des Benutzers an den Benutzer; **dadurch gekennzeichnet, dass** die Vorgänge Folgendes umfassen:
Anfordern einer Rückmeldung des Benutzers über die Art der Atmung oder den Zustand des Benutzers;
Empfangen (1216) einer Rückmeldung von dem Benutzer über die Art der Atmung oder den Zustand des Benutzers; und
Aktualisieren (1218) des trainierten maschinellen Lernmodells auf Grundlage der von dem Benutzer empfangenen Rückmeldung.

9. Nicht transitorisches maschinenlesbares Speichermedium nach Anspruch 8, wobei die beobachteten atembezogenen Parameter die Audiofrequenzverteilungsmerkmale eines Einatmens oder eines Ausatmens enthalten.

10. Nicht transitorisches maschinenlesbares Speichermedium nach Anspruch 8, wobei das trainierte maschinelle Lernmodell die Art der Atmung oder den Zustand des Benutzers mindestens teilweise durch Vergleichen der beobachteten atembezogenen Parameter mit atembezogenen Referenzparametern bestimmt.

11. Nicht transitorisches maschinenlesbares Speichermedium nach Anspruch 10, wobei die Vorgänge ferner Folgendes beinhalten:
Bestimmen einer Genauigkeitsbewertungsziffer aus der bestimmten Art der Atmung oder einem Zustand des Benutzers auf Grundlage darauf, wie genau eine beobachtete Respirationsrate des Benutzers mit einer angeforderten oder gewünschten Respirationsrate übereinstimmt; und
Bereitstellen einer Ausgabe der Genauigkeitsbewertungsziffer für den Benutzer.

12. Nicht transitorisches maschinenlesbares Speichermedium nach Anspruch 11, wobei die Genauigkeitsbewertungsziffer ferner auf einer beobachteten Zeitspanne basiert, in der die beobachtete Respirationsrate innerhalb eines Schwellenwertes der angeforderten oder gewünschten Respirationsrate liegt.

13. System, das Folgendes umfasst:
einen oder mehrere Prozessoren (1304); und
ein oder mehrere maschinenlesbare Medien, die Anweisungen speichern, die, wenn sie von dem einen oder den mehreren Prozessoren (1304) ausgeführt werden, das System veranlassen, Vorgänge zum Überwachen der Atmung eines Benutzers durchzuführen, wobei die Vorgänge Folgendes umfassen:
Empfangen (1206) einer Vielzahl von Audiosignalen von einer Vielzahl von Mikrofonen;
Strahlausbilden (1206) der Vielzahl von Audiosignalen in ein strahlausgebildetes Audiosignal;
Bestimmen (1210) beobachteter atembezogener Parameter aus dem strahlausgebildeten Audiosignal, wobei der Schritt des Bestimmens atembezogener Parameter Folgendes umfasst:
Bestimmen (1212) einer Art der Atmung oder eines Zustands des Benutzers aus den beobachteten atembezogenen Parametern unter Verwendung eines trainierten maschinellen Lernmodells;
Bereitstellen (1214) einer Ausgabe der Art der Atmung oder eines Zustands des Benutzers an den Benutzer; **dadurch gekennzeichnet, dass** die Vorgänge Folgendes umfassen:
Anfordern einer Rückmeldung des Benutzers über die Art der Atmung oder den Zustand des Benutzers;
Empfangen (1216) einer Rückmeldung von dem Benutzer über die Art der Atmung oder den Zustand des Benutzers; und
Aktualisieren (1218) des trainierten maschinellen Lernmodells auf Grundlage der von dem Benutzer empfangenen Rückmeldung.

14. System nach Anspruch 13, wobei die beobachteten atembezogenen Parameter die Audiofrequenzverteilungsmerkmale eines Einatmens oder eines Ausatmens enthalten.

15. System nach Anspruch 13, wobei die Vorgänge ferner Folgendes umfassen:
Ableiten einer Atmungsbewertungsziffer aus der bestimmten Art der Atmung oder einem Zustand des Benutzers, wobei die Atmungsbewertungsziffer auf einer Kombination basiert, wie genau eine beobachtete Respiration des Benutzers mit einer angeforderten oder gewünschten Respirationsrate und einer beobachteten Zeitspanne, in der die beobachtete Respirationsrate innerhalb eines Schwellenwerts der angeforderten oder gewünschten Respirationsrate liegt, übereinstimmt.

## Revendications

1. Procédé de surveillance de la respiration d'un utilisateur, exécuté par un ou plusieurs processeurs, le procédé comprenant :
la réception (1206) d'une pluralité de signaux audio provenant d'une pluralité de microphones ;
l'application d'une formation de faisceau (1206) à la pluralité de signaux audio afin d'obtenir un signal audio formé par formation de faisceau ;
la détermination (1210) de paramètres observés liés à l'haleine à partir du signal audio formé par formation de faisceau, ladite étape de détermination des paramètres liés à l'haleine comprenant :
la détermination (1212) d'une nature de la respiration ou d'un état de l'utilisateur à partir des paramètres observés liés à l'haleine en utilisant un modèle d'apprentissage automatique entraîné ;
la fourniture (1214) à l'utilisateur d'une sortie indiquant la nature de la respiration ou un état de l'utilisateur ;
**caractérisé en ce que** le procédé comprend en outre :
la demande d'un retour de l'utilisateur sur la nature de la respiration ou l'état de l'utilisateur ;
la réception (1216) d'un retour de l'utilisateur sur la nature de la respiration ou l'état de l'utilisateur ; et
la mise à jour (1218) du modèle d'apprentissage automatique entraîné en fonction du retour reçu de l'utilisateur.

2. Procédé selon la revendication 1, dans lequel les paramètres observés liés à l'haleine comprennent des caractéristiques de distribution de fréquences audio d'une inspiration ou d'une expiration.

3. Procédé selon la revendication 1, dans lequel le modèle d'apprentissage automatique entraîné détermine la nature de la respiration ou l'état de l'utilisateur au moins en partie en comparant les paramètres observés liés à l'haleine avec des paramètre de référence liés à l'haleine.

4. Procédé selon la revendication 3, dans lequel les paramètres de référence liés à l'haleine sont des paramètres historiques liés à l'haleine pour l'utilisateur.

5. Procédé selon la revendication 3, dans lequel les paramètres observés liés à l'haleine comprennent des caractéristiques de distribution de fréquences audio d'une inspiration ou d'une expiration.

6. Procédé selon la revendication 3, dans lequel les paramètres de référence liés à l'haleine sont des paramètres démographiques liés à l'haleine basés au moins sur l'âge et le sexe.

7. Procédé selon la revendication 3, comprenant en outre :
la détermination, à partir de la nature déterminée de la respiration ou d'un état de l'utilisateur, d'un score de précision basé sur la mesure dans laquelle une respiration observée de l'utilisateur correspond étroitement à un rythme respiratoire demandé ou souhaité ; et
la fourniture à l'utilisateur d'une sortie indiquant le score de précision.

8. Support d'enregistrement non transitoire lisible par machine comprenant des instructions qui, lorsqu'elles sont exécutées par un ou plusieurs processeurs d'une machine, amènent la machine à effectuer des opérations pour la surveillance de la respiration d'un utilisateur, les opérations comprenant :
la réception (1206) d'une pluralité de signaux audio provenant d'une pluralité de microphones ;
l'application d'une formation de faisceau (1206) à la pluralité de signaux audio afin d'obtenir un signal audio formé par formation de faisceau ;
la détermination (1210) de paramètres observés liés à l'haleine à partir du signal audio formé par formation de faisceau, ladite étape de détermination des paramètres liés à l'haleine comprenant :
la détermination (1212) d'une nature de la respiration ou d'un état de l'utilisateur à partir des paramètres observés liés à l'haleine en utilisant un modèle d'apprentissage automatique entraîné ;
la fourniture (1214) à l'utilisateur d'une sortie indiquant la nature de la respiration ou un état de l'utilisateur, **caractérisé en ce que** les opérations comprennent :
la demande d'un retour de l'utilisateur sur la nature de la respiration ou l'état de l'utilisateur ;
la réception (1216) d'un retour de l'utilisateur sur la nature de la respiration ou l'état de l'utilisateur ; et
la mise à jour (1218) du modèle d'apprentissage automatique entraîné en fonction du retour reçu de l'utilisateur.

9. Support d'enregistrement non transitoire lisible par machine selon la revendication 8, dans lequel les paramètres observés liés à l'haleine comprennent des caractéristiques de distribution de fréquences audio d'une inspiration ou d'une expiration.

10. Support d'enregistrement non transitoire lisible par machine selon la revendication 8, dans lequel le modèle d'apprentissage automatique entraîné détermine la nature de la respiration ou l'état de l'utilisateur au moins en partie en comparant les paramètres observés liés à l'haleine avec des paramètre de référence liés à l'haleine.

11. Support d'enregistrement non transitoire lisible par machine selon la revendication 10, dans lequel les opérations comprennent en outre :
la détermination, à partir de la nature déterminée de la respiration ou d'un état de l'utilisateur, d'un score de précision basé sur la mesure dans laquelle un rythme respiratoire observé de l'utilisateur correspond étroitement à un rythme respiratoire demandé ou souhaité ; et
la fourniture à l'utilisateur d'une sortie indiquant le score de précision.

12. Support d'enregistrement non transitoire lisible par machine selon la revendication 11, dans lequel le score de précision est en outre basé sur une durée observée pendant laquelle le rythme respiratoire observé se situe dans les limites d'une valeur seuil du rythme respiratoire demandé ou souhaité.

13. Système comprenant :
un ou plusieurs processeurs (1304) ; et
un ou plusieurs supports lisibles par machine sur lesquels sont enregistrées des instructions qui, lorsqu'elles sont exécutées par les un ou plusieurs processeurs (1304), amènent le système à effectuer des opérations pour la surveillance de la respiration d'un utilisateur, les opérations comprenant :
la réception (1206) d'une pluralité de signaux audio provenant d'une pluralité de microphones ;
l'application d'une formation de faisceau (1206) à la pluralité de signaux audio afin d'obtenir un signal audio formé par formation de faisceau ;
la détermination (1210) de paramètres observés liés à l'haleine à partir du signal audio formé par formation de faisceau, ladite étape de détermination des paramètres liés à l'haleine comprenant :
la détermination (1212) d'une nature de la respiration ou d'un état de l'utilisateur à partir des paramètres observés liés à l'haleine en utilisant un modèle d'apprentissage automatique entraîné ;
la fourniture (1214) à l'utilisateur d'une sortie indiquant la nature de la respiration ou un état de l'utilisateur ; **caractérisé en ce que** les opérations comprennent :
la demande d'un retour de l'utilisateur sur la nature de la respiration ou l'état de l'utilisateur ;
la réception (1216) d'un retour de l'utilisateur sur la nature de la respiration ou l'état de l'utilisateur ; et
la mise à jour (1218) du modèle d'apprentissage automatique entraîné en fonction du retour reçu de l'utilisateur.

14. Système selon la revendication 13, dans lequel les paramètres observés liés à l'haleine comprennent des caractéristiques de distribution de fréquences audio d'une inspiration ou d'une expiration.

15. Système selon la revendication 13, dans lequel les opérations comprennent en outre :
la dérivation, à partir de la nature déterminée de la respiration ou d'un état de l'utilisateur, d'un score respiratoire, le score respiratoire étant basé sur une combinaison de la mesure dans laquelle une respiration observée de l'utilisateur correspond étroitement à un rythme respiratoire demandé ou souhaité et d'une durée observée pendant laquelle le rythme respiratoire observé se situe dans les limites d'une valeur seuil du rythme respiratoire demandé ou souhaité.
